# EUROPEAN PATENT SPECIFICATION

(11) **EP 3 448 369 B1**
(45) Date of publication and mention of the grant of the patent: **25.06.2025**
(21) Application number: 17790539.5
(22) Date of filing: 28.04.2017
(51) Int. Cl.: A61K 31/713, A61K 31/05, A61P 3/10, A61P 9/12, G01N 33/68, A61K 45/06

(54) **INHIBITION OR DEGRADATION OF HDAC11 FOR TREATING OBESITY, HYPERLIPIDEMIA, TYPE II DIABETES, METABOLIC SYNDROME OR INSULIN RESISTANCE, FOR PROMOTING WEIGHT LOSS, AND/OR AMELIORATING OR PREVENTING WEIGHT GAIN**
HEMMUNG ODER ABBAU VON HDAC11 ZUR BEHANDLUNG VON FETTLEIBIGKEIT, HYPERLIPIDÄMIE, TYP-II-DIABETES, METABOLISCHEM SYNDROM ODER INSULINRESISTENZ, UND ZUR FÖRDERUNG DER GEWICHTSABNAHME UND/ODER ZUR LINDERUNG ODER VERHINDERUNG EINER GEWICHTSZUNAHME
INHIBITION OU DÉGRADATION DE HDAC11 POUR LE TRAITEMENT DE L'OBÉSITÉ, DE L'HYPERLIPIDÉMIE, DU DIABÈTE DE TYPE II, DU SYNDROME MÉTABOLIQUE OU DE LA RÉSISTANCE À L'INSULINE, ET POUR FAVORISER LA PERTE DE POIDS ET/OU AMÉLIORER OU PRÉVENIR LA PRISE DE POIDS

(30) Priority: 29.04.2016 US 201662329843 P
(43) Date of publication of application: 06.03.2019
(73) Proprietor: The Regents of The University of Colorado, A Body Corporate, Denver, CO 80203 (US)
(72) Inventor: MCKINSEY, Timothy A., Broomfield Colorado 80020 (US); BAGCHI, Rushita, Aurora Colorado 80011 (US); FERGUSON, Bradley S., Reno NV 89509 (US); STRATTON, Matthew S., Worthington, OH 43085 (US)
(74) Representative: Grünecker Patent- und Rechtsanwälte PartG mbB
(86) International application number: PCT/US2017/030118
(87) International publication number: WO 2017/190001

(56) References cited:
- WO-A1-2014/018979
- WO-A1-2014/096386
- WO-A2-2013/149258
- US-A1- 2011 182 888
- US-A1- 2014 314 788
- US-A1- 2016 304 462
- DATABASE BIOSIS [online] BIOSCIENCES INFORMATION SERVICE, PHILADELPHIA, PA, US; December 2015 (2015-12-01), HUANG XIAO-DI ET AL: "A Role of HDAC11 in Adipogenic Differentiation among Mesenchymal Stem Cells C3H10TI/2 Expressed HDACs", XP002795741, Database accession no. PREV201600175543
- WEI XU ET AL: "Metabolism is regulated by protein acetylation", FRONTIERS OF BIOLOGY IN CHINA ; SELECTED PUBLICATIONS FROM CHINESE UNIVERSITIES, HIGHER EDUCATION PRESS, BE, vol. 6, no. 3, 5 June 2011 (2011-06-05), pages 213 - 218, XP019912174, ISSN: 1673-3622, DOI: 10.1007/S11515-011-1126-6

## Description

### BACKGROUND OF THE DISCLOSURE

Metabolic syndrome, or metabolic disease, is the term used to define a syndrome associated with at least three of five of the following medical conditions: abdominal (central) obesity, elevated blood pressure, elevated fasting plasma glucose, high serum triglycerides, and low high-density lipoprotein (HDL) levels. An individual with metabolic syndrome is at higher risk of developing cardiovascular disease and diabetes. About a third of the U.S. population is thought to have metabolic syndrome, and some researchers believe that metabolic syndrome and prediabetes may be the same disorder but diagnosed using distinct sets of biomarkers.

Metabolic syndrome is thought to be associated with dysregulated energy utilization and storage. The main sign of metabolic syndrome is central or visceral obesity, with adipose tissue accumulation particularly around the waist and trunk. Other signs of metabolic syndrome include high blood pressure, decreased fasting serum HDL cholesterol, elevated fasting serum triglyceride level (VLDL triglyceride), impaired fasting glucose, insulin resistance, or prediabetes. Associated conditions include hyperuricemia, fatty liver (which may progress to nonalcoholic fatty liver disease), polycystic ovarian syndrome (in women), erectile dysfunction (in men), and acanthosis nigricans.

There are two types of fat (adipose tissue) in animals: white adipose tissue (WAT) and brown adipose tissue (BAT). BAT is especially abundant in newborns and hibernating mammals, and owes its color to a higher number of iron-containing mitochondria and capillaries. In newborns, BAT helps avoid hypothermia through heat-generating ATP uncoupling reactions. It was commonly thought that adults lacked BAT, which had been converted to WAT as the mammal aged and/or gained weight. However, deposits of BAT have been identified in adult mammals. It is now known that activation of BAT reduces plasma triglyceride and cholesterol levels and attenuates diet-induced atherosclerosis development.

There is thus a need in the art for compositions and methods that treat, revert, and/or prevent metabolic syndrome in a mammal. There is a further need in the art for compositions and methods that treat, revert and/or prevent insulin resistance in a mammal. There is further need in the art for compositions and methods that promote increase in % BAT, as a function of total adipose tissue, in a mammal. The present disclosure addresses and fulfills these needs.

### BRIEF SUMMARY OF THE DISCLOSURE

The invention provides a composition for use in a method a method of treating, reverting, and/or preventing obesity, hyperlipidemia, type II diabetes, metabolic syndrome or insulin resistance in a subject in need thereof as is defined in claim 1. The invention further provides a non-therapeutic use for promoting weight loss, and/or ameliorating or preventing weight gain as defined in claim 2.

In certain embodiments, the subject has at least one condition selected from the group consisting of obesity, elevated blood pressure, elevated fasting plasma glucose, high serum triglyceride levels, and low high-density lipoprotein (HDL) levels.

In certain embodiments, the subject has type II diabetes.

According to claim 1, the composition comprises at least one agent selected from the group consisting of an antibody, siRNA, ribozyme, antisense RNA, modified antisense RNA, small molecule, and any combinations thereof.

In certain embodiments, the antibody comprises an antibody selected from the group consisting of a polyclonal antibody, monoclonal antibody, humanized antibody, synthetic antibody, heavy chain antibody, human antibody, biologically active fragment of an antibody, and any combinations thereof.

In certain embodiments, the composition is administered to the subject by an inhalational, oral, rectal, vaginal, parenteral, topical, transdermal, pulmonary, intranasal, buccal, ophthalmic, intrathecal, intracranial, or intravenous route of administration.

In certain embodiments, the composition is co-administered with at least one additional agent that treats and/or prevents metabolic syndrome, or promotes weight loss. In other embodiments, the at least one additional agent comprises a weight loss promoter or anti-diabetic medicament. In yet other embodiments, the subject is a mammal. In yet other embodiments, the mammal is human.

In certain embodiments, the kit may be empoyed that comprises an instruction manual reciting how to perform at least one method using the pharmaceutical composition.

### BRIEF DESCRIPTION OF THE DRAWINGS

For the purpose of illustrating the disclosure, there are depicted in the drawings certain embodiments of the invention. However, the disclosure is not limited to the precise arrangements and instrumentalities of the embodiments depicted in the drawings.
FIG. 1 illustrates body weight results obtained with HDAC11 (histone deacetylase 11) knockout (KO) mice, as compared to the counterpart wild-type (WT) animals.
FIGs. 2A-2C illustrate results indicating the increased adipogenic differentiation in HDAC11 KO mouse embryonic fibroblasts (MEFs).
FIGs. 3A-3B illustrate results indicating the increased adipogenic differentiation in HDAC11 KO MEFs.
FIG. 4 illustrates a non-limiting 3T3-L1 differentiation workflow.
FIGs. 5A-5B illustrate results relating to 3T3-L1 differentiation using the workflow illustrated in FIG. 4.
FIG. 6 illustrates a set of protein gel images showing expression of adipogenic markers during 3T3-L1 differentiation.
FIG. 7 comprises a graph illustrating downregulation of HDAC11 during the course of differentiation in 3T3-L1 cells.
FIG. 8 illustrates a set of protein gel images showing HDAC11 lentivirus overexpression in 3T3-L1 pre-adipocytes.
FIGs. 9A-9B illustrate results showing that HDAC11 overexpression represses adipogenic differentiation in 3T3-L1 cells.
FIG. 10 comprises a graph illustrating the finding that HDAC11 overexpression reduces lipid accumulation during adipogenesis.
FIG. 11 illustrates a set of protein gel images showing that HDAC11 overexpression blunts adipogenesis in 3T3-L1 cells.
FIG. 12 comprises a set of bar graphs illustrating that HDAC11 attenuates PPARγ and C/EBPα promoter transactivation.
FIG. 13 comprises a set of phase contrast images illustrating that HDAC11 knockdown enhances adipogenic differentiation in MEFs.
FIG. 14 comprises a set of Oil red O-stained images illustrating that HDAC11 knockdown enhances adipogenic differentiation in MEFs.
FIG. 15 comprises a bar graph illustrating that HDAC11 knockdown augments lipid accumulation during adipogenesis in MEFs.
FIG. 16 comprises a set of protein gel images illustrating that HDAC11 knockdown enhances adipogenic differentiation in MEFs.
FIG. 17 comprises a set of images illustrating certain roles that BRD2 plays in adipogenesis.
FIG. 18 comprises a non-limiting illustration of certain proteins involved in gene transcription.
FIG. 19 illustrates results indicating that BRD2 expression is downregulated during adipogenesis in 3T3-L1 cells.
FIG. 20 illustrates a set of protein gel images supporting the finding that HDAC11 overexpression drives BRD2 expression in 3T3-L1 cells.
FIG. 21 comprises a protein gel image illustrating the finding that HDAC11 physically interacts with BRD2.
FIG. 22 comprises a set of protein gel images illustrating results obtained from BRD2 knockdown in 3T3-L1 pre-adipocytes using lentiviral delivery.
FIG. 23 comprises a set of protein gel images illustrating the finding that *Brd2* gene knockdown partially rescues HDAC11-mediated effects on adipogenesis.
FIG. 24 comprises selected images illustrating the finding that *Brd2* gene knockdown partially rescues HDAC11-mediated effects on adipogenesis.
FIG. 25 comprises a bar graph illustrating the finding that *Brd2* gene knockdown partially rescues HDAC11-mediated effects on adipogenesis.
FIG. 26 comprises a set of bar graphs illustrating the finding that HDAC11 KO mice have increased body mass, increased lean mass and similar fat mass as corresponding WT inter mates.
FIG. 27 comprises a set of bar graphs illustrating the finding that HDAC11 KO mice have increased brown fat mass as corresponding WT inter mates. eWAT = epididymal white adipose tissue; ingWAT = inguinal white adipose tissue; iBAT = interscapular brown adipose tissue.
FIG. 28 comprises a graph illustrating the finding that HDAC11 KO mice maintain higher core body temperature than WT in response to cold challenge. WT and HDAC11 KO mice (n=4 per group) were placed in a 8-chamber Oxymax indirect calorimetry system (CLAMS) with chamber temperature set at 4°C for 24 hours. Core body temperature was measured using a rectal probe thermometer at the indicated time points. The KO mice maintained significantly higher body temperature throughout the duration of cold exposure. *p<0.05 vs WT mice.
FIGs. 29A-29B comprise a set of bar graphs illustrating a metabolic profile of HDAC11^{-/-}mice in response to cold challenge. WT and HDAC11 KO mice (n=4 per group) were placed in a 8-chamber Oxymax indirect calorimetry system (CLAMS) with chamber temperature set at 4°C for 24 hours. Activity was measured using laser beam breaks (FIG. 29A). Oxygen consumption (VO₂; FIG. 29B), metabolic rate (MR; FIG. 29C) and respiratory exchange ratio (RER; FIG. 29D was also measured. The KO mice exhibited significantly increased activity, VO₂, MR and RER. *p<0.05 vs WT mice.
FIGs. 30A-30B comprise a set of bar graphs illustrating thermogenic gene expression of HDAC11^{-/-} mice in response to cold challenge. RNA samples from brown adipose tissue (BAT; FIG. 30A) and inguinal white adipose tissue (ingWAT; FIG. 30B) samples of WT and HDAC11 KO mice (n=4 per group) exposed to 4°C for 24h were reverse transcribed to cDNA and subjected to qPCR for Ucp1 and Pgc1a. Tissue samples from mice maintained at room temperature (RT) in the vivarium were identically processed. Gene expression values were calculated using the 2^{ΔΔCt} method. *p<0.05 vs WT RT; #p<0.05 vs WT 4°C.

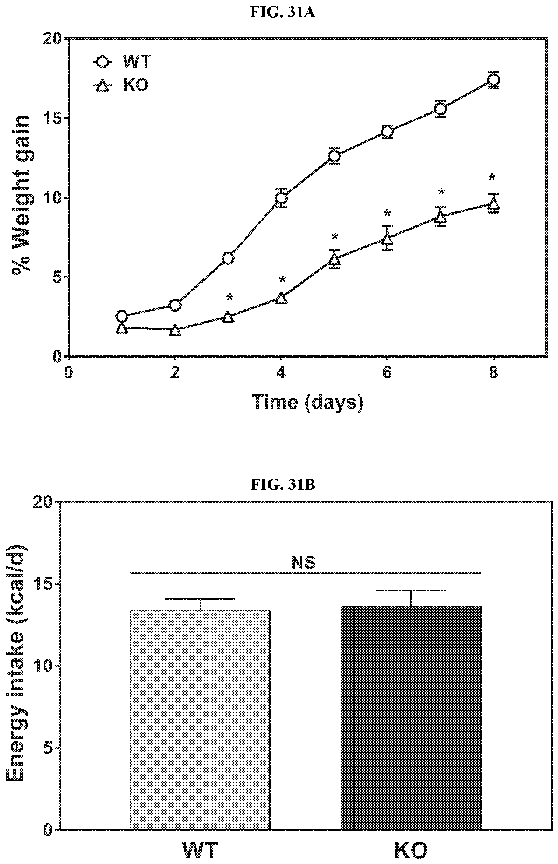

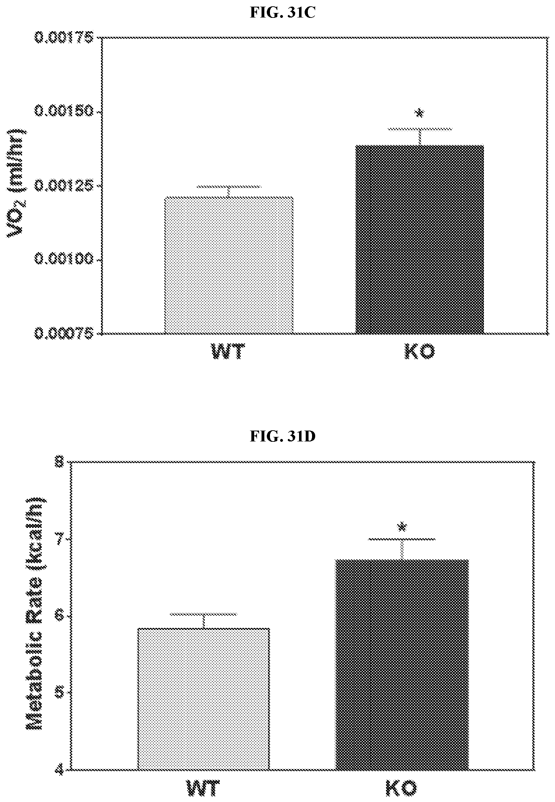

FIGs. 31A-31B comprise a set of graph and bar graphs illustrating metabolic profile of HDAC11^{-/-} mice in response to acute high calorie diet. WT and HDAC11 KO mice (n=4 per group) were placed in a 8-chamber Oxymax indirect calorimetry system (CLAMS) with chamber temperature set at thermoneutrality for 8 days. The mice were exposed to a high fat diet (60% fat; Western diet) during this time. Body weights were recorded daily and daily % weight gain (relative to initial body weight) was calculated (FIG. 31A). Energy intake (FIG. 31B) was similar between both WT and KO groups. Oxygen consumption (VO₂; FIG. 31C) and metabolic rate (FIG. 31D) were also measured. *p<0.05 vs WT mice.

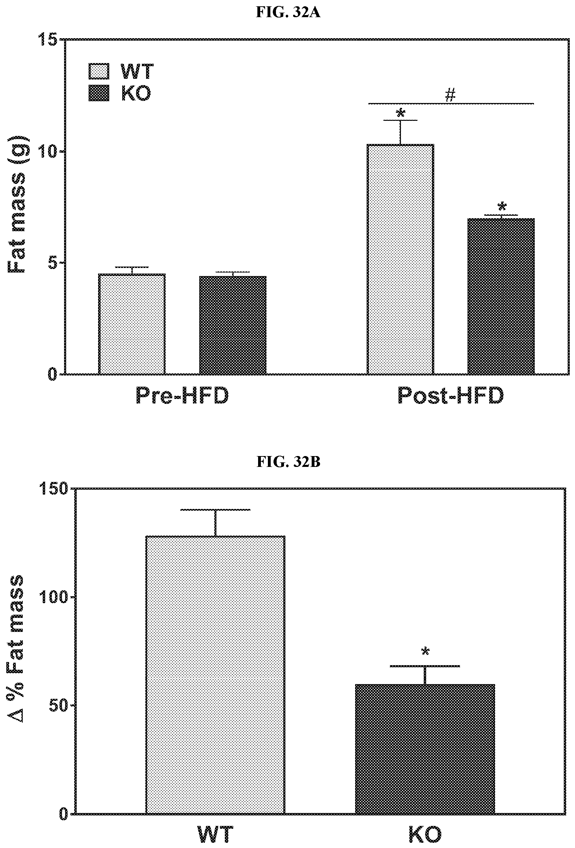

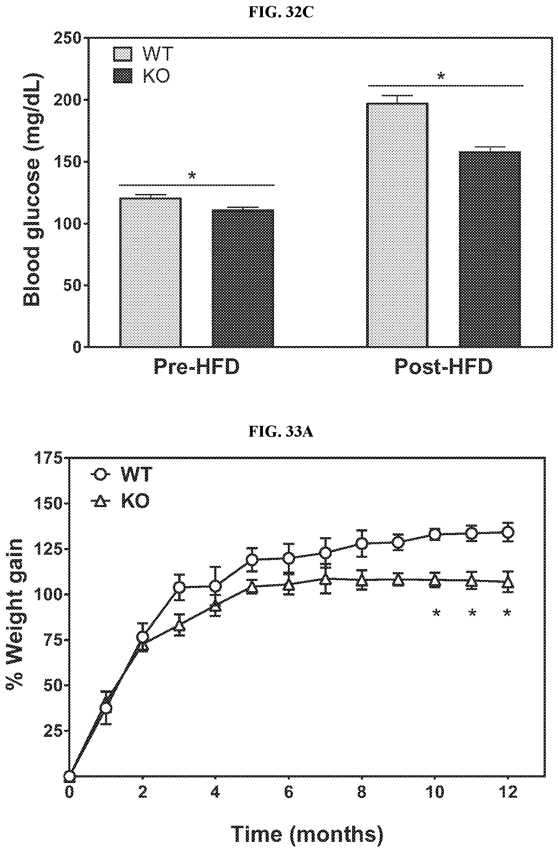

FIGs. 32A-32C comprise a set of bar graphs illustrating WT and HDAC11 KO mice (n=4 per group) were placed in a 8-chamber Oxymax indirect calorimetry system (CLAMS) with chamber temperature set at thermoneutrality for 8 days. The mice were fed a high fat diet (60% fat; Western diet) during this time. Fat mass of WT and KO mice were measured using QMRI prior and post diet (FIG. 32A). % change in fat mass was calculated relative to initial fat mass values (FIG. 32B). Blood glucose was measured using Bayer Contour glucometer at the beginning and end of the diet (FIG. 32C). N=4; *p<0.05 vs WT/ WT-PreHFD.

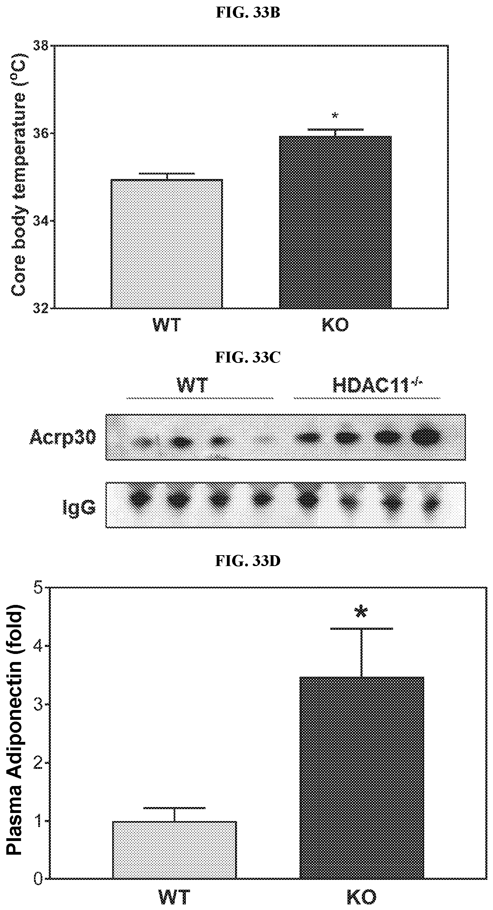

FIGs. 33A-33D comprise a set of graph and bar graphs illustrating embodiments relating to HDAC11^{-/-} mice on chronic high calorie diet. WT (n=4) and HDAC11 KO (n=5) mice were fed high fat diet (60% fat; Western diet) for 12 months. Body weights were recorded at regular intervals and monthly % weight gain (relative to initial body weight) was calculated and plotted (FIG. 33A). Core body temperature was measured using a rectal probe thermometer in unanesthetized mice at the end of the study (FIG. 33B). Plasma samples collected by cardiac puncture from WT and KO mice at the time of sacrifice were resolved on a 4-20% precast gel (Biorad) and subjected to western blotting for adiponectin (FIGs. 33A-33D). Mouse IgG was used as loading control. N=4; *p<0.05 vs WT.
FIG. 34 comprises a graph illustrating blood glucose levels in HDAC11^{-/-} mice on chronic high calorie diet. WT (n=4) and HDAC11 KO (n=5) mice were fed high fat diet (60% fat; Western diet) for 12 months. Glucose Tolerance Test was performed to examine glucose intolerance in WT and KO mice in response to chronic high fat diet. Mice were starved for 16hrs, followed by i.p. injection of glucose (2g/kg). Blood glucose was measured at the indicated time points. *p<0.05 vs WT.
FIG. 35 comprises a set of images illustrating liver tissues of HDAC11^{-/-} mice on chronic high calorie diet. WT (n=4) and HDAC11 KO (n=5) mice were fed high fat diet (60% fat; Western diet) for 12 months. Liver tissues were fixed in 10% formalin and processed for paraffin embedding. 5µm thick sections were cut and stained with hematoxylin and eosin. Images were obtained using an inverted microscope (Zeiss) with 40X objective lens. Scale bar: 40µM. Arrows are used to indicate lipid/ fat droplets.

### DETAILED DESCRIPTION OF THE DISCLOSURE

The present invention is defined in the claims. The teaching of the invention as claimed relates, in one aspect, to the unexpected discovery that inhibitors of HDAC11 expression and/or activity can be used to treat, revert, and/or prevent metabolic syndrome in a mammal. Inhibitors of HDAC11 expression and/or activity increase, or prevent decrease of, brown adipose deposits in the mammal. Further, inhibitors of HDAC11 expression and/or activity treat, revert and/or prevent insulin resistance in a mammal. Moreove, inhibitors of HDAC11 expression and/or activity accelerate, or prevent decrease of, metabolic rate in a mammal. Furthermore, inhibitors of HDAC11 expression and/or activity promote weight loss and/or ameliorate or prevent weight gain in a mammal.

HDAC11 is a 39 kDA protein that is highly expressed in the brain, heart, skeletal muscle, kidney and testis. Little is known about target gene regulation by HDAC11. As demonstrated herein, HDAC11 KO mice were found to have increased body weight compared to the WT animals, but with beneficial brown fat deposits.

As demonstrated herein, HDAC11 deletion in mice leads to enhanced production of metabolically active brown adipose tissue (BAT). Further, HDAC11 overexpression in cell culture blocks white adipose tissue (WAT) production (or white fat adipogenesis). Compounds and/or compositions that inhibit HDAC11 expression and/or activity, and/or promote HDAC11 degradation, sustain or increase BAT activity/function in a mammal, and thus are useful for the treatment of obesity, hyperlipidemia and/or type 2 diabetes in the mammal.

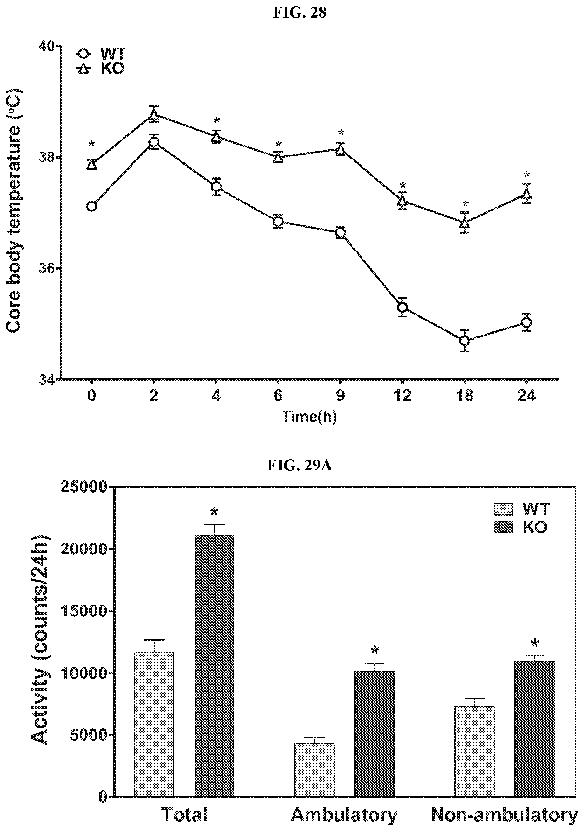

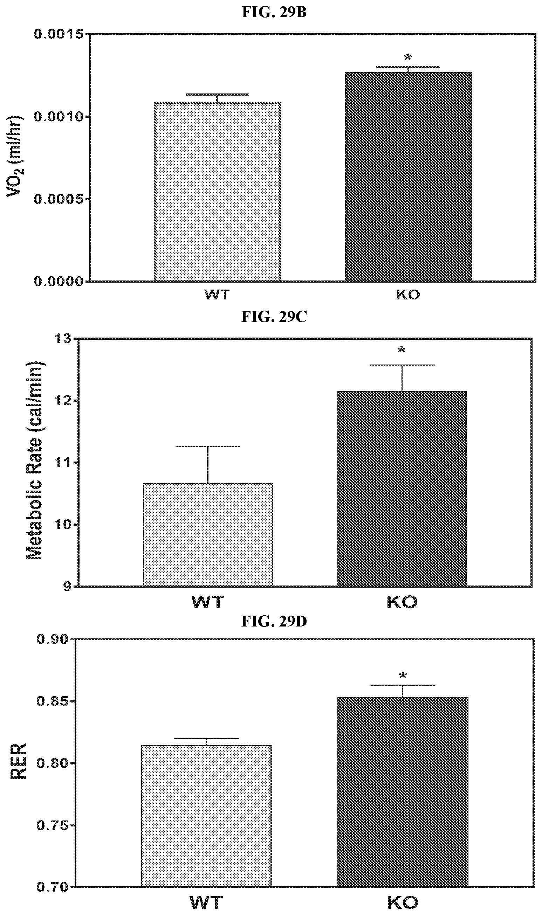

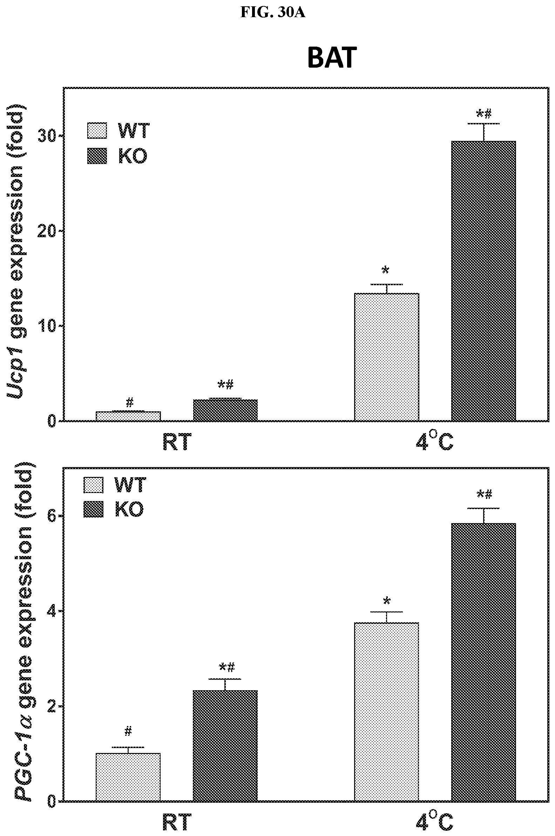

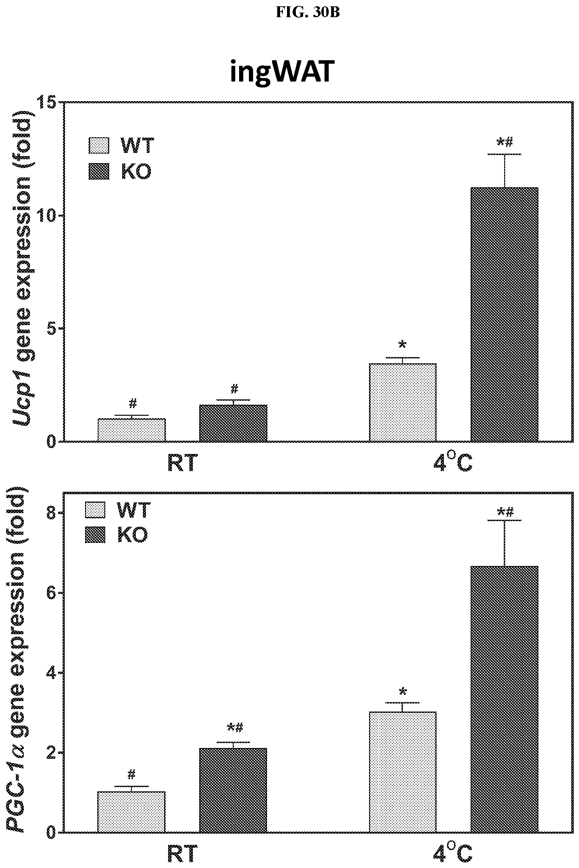

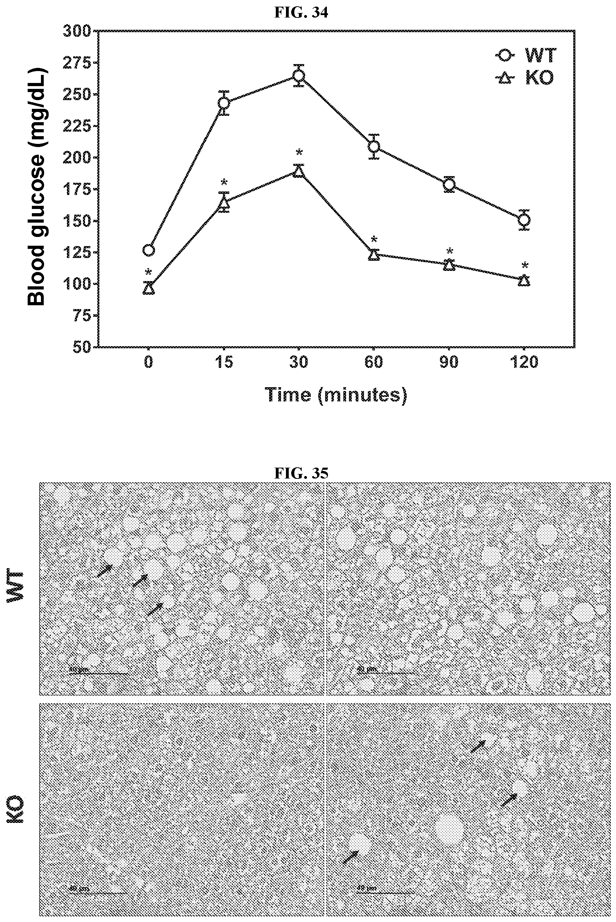

As demonstrated herein, HDAC11 KO mice maintain a higher core body temperature than the WT mice in response to cold challenge (FIG. 28). In response to a cold challenge, HDAC11 KO mice have statistically higher activity, oxygen consumption (VO₂), metabolic rate (MR) and respiratory exchange ratio as compared to WT mice (FIGs. 29A-29D). In response to a cold challenge, HDAC11 KO mice have higher expression of brown adipose tissue (BAT) and inguinal white adipose tissue (ingWAT) than WT mice (FIGs. 30A-30B). In response to an acute high calorie diet, HDAC11 KO mice gain less weight, and have higher oxygen consumption (VO₂) and metabolic rate, than WT mice with the same energy (caloric) intake. In response to an acute high calorie diet, HDAC11 KO mice have lower overall increase in fat mass and lower increase in blood glucose (FIGs. 32A-32-C). In response to a chronic high calorie diet, HDAC11 KO mice develop higher core body temperature and higher adiponectin levels than WT mice (FIGs. 33A-33D). In response to a chronic high calorie diet, HDAC11 KO mice have lower blood glucose levels than WT mice (FIG. 34). In response to a chronic high calorie diet, HDAC11 KO mice develop fewer/smaller lipid or fat droplets in the liver (FIG. 35).

In one aspect, the present invention provides a composition that inhibits HDAC11 expression and/or activity, or induces HDAC11 degradation for use in (i) a method of treating, reverting, and/or preventing obesity, hyperlipidemia, type II diabetes, metabolic syndrome or insulin resistance in a subject in need thereof, wherein the composition comprises at least one agent selected from the group consisting of an antibody, siRNA, ribozyme, antisense RNA, modified antisense RNA, small molecule, and any combinations thereof.

In another aspect, the present invention provides the non-therapeutic use of a composition that inhibits HDAC11 expression and/or activity, or induces HDAC11 degradation for promoting weight loss, and/or ameliorating or preventing weight gain, wherein the composition comprises at least one agent selected from the group consisting of an antibody, siRNA, ribozyme, antisense RNA, modified antisense RNA, small molecule, and any combinations thereof.

### Definitions

As used herein, each of the following terms have the meaning associated with it in this section.

Unless defined otherwise, all technical and scientific terms used herein generally have the same meaning as commonly understood by one of ordinary skill in the art to which this disclosure belongs. Generally, the nomenclature used herein and the laboratory procedures in cell culture, molecular genetics and chemistry are those well-known and commonly employed in the art.

As used herein, the articles "a" and "an" refer to one or to more than one *(i.e.,* to at least one) of the grammatical object of the article. By way of example, "an element" means one element or more than one element.

As used herein, the term "about" will be understood by persons of ordinary skill in the art and will vary to some extent on the context in which it is used. As used herein when referring to a measurable value such as an amount, a temporal duration, and the like, the term "about" is meant to encompass variations of ±20% or ±10%, ±5%, ±1 %, or ±0.1 % from the specified value, as such variations are appropriate to perform the disclosed methods.

A disease or disorder is "alleviated" if the severity of a symptom of the disease or disorder, the frequency with which such a symptom is experienced by a patient, or both, is reduced.

The term "antibody," as used herein, refers to an immunoglobulin molecule that specifically binds with an antigen. Antibodies may be intact immunoglobulins derived from natural sources or from recombinant sources and may be immunoreactive portions of intact immunoglobulins. Antibodies are typically tetramers of immunoglobulin molecules. The antibodies in the present disclosure may exist in a variety of forms including, for example, polyclonal antibodies, monoclonal antibodies, Fv, Fab and F(ab)2, as well as single chain antibodies and humanized antibodies (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, New York; Houston et al., 1988, Proc Natl Acad Sci USA 85:5879-5883; Bird et al., 1988, Science 242:423-426).

The term "antigen" or "Ag" as used herein is defined as a molecule that provokes an immune response. This immune response may involve either antibody production, or the activation of specific immunologically-competent cells, or both. The skilled artisan will understand that any macromolecule, including virtually all proteins or peptides, can serve as an antigen. Furthermore, antigens can be derived from recombinant or genomic DNA. A skilled artisan will understand that any DNA, which comprises a nucleotide sequences or a partial nucleotide sequence encoding a protein that elicits an immune response therefore encodes an "antigen" as that term is used herein. Furthermore, one skilled in the art will understand that an antigen need not be encoded solely by a full length nucleotide sequence of a gene. It is readily apparent that the present disclosure includes, but is not limited to, the use of partial nucleotide sequences of more than one gene and that these nucleotide sequences are arranged in various combinations to elicit the desired immune response. Moreover, a skilled artisan will understand that an antigen need not be encoded by a "gene" at all. It is readily apparent that an antigen can be generated synthesized or can be derived from a biological sample. Such a biological sample can include, but is not limited to a tissue sample, a tumor sample, a cell or a biological fluid.

"Antisense" refers particularly to the nucleic acid sequence of the non-coding strand of a double stranded DNA molecule encoding a polypeptide, or to a sequence which is substantially homologous to the non-coding strand. As defined herein, an antisense sequence is complementary to the sequence of a double stranded DNA molecule encoding a polypeptide. It is not necessary that the antisense sequence be complementary solely to the coding portion of the coding strand of the DNA molecule. The antisense sequence may be complementary to regulatory sequences specified on the coding strand of a DNA molecule encoding a polypeptide, which regulatory sequences control expression of the coding sequences.

As used herein, the term "Brd2" or "BRD2" (SEQ ID NO:1) refers to Bromodomain-containing protein 2, which in humans is encoded by the *BRD2* gene and in non-humans is encoded by the *Brd2* gene:

10 20 30 40 50
MLQNVTPHNK LPGEGNAGLL GLGPEAAAPG KRIRKPSLLY EGFESPTMAS
60 70 80 90 100
VPALQLTPAN PPPPEVSNPK KPGRVTNQLQ YLHKVVMKAL WKHQFAWPFR
110 120 130 140 150
QPVDAVKLGL PDYHKIIKQP MDMGTIKRRL ENNYYWAASE CMQDFNTMFT
160 170 180 190 200
NCYIYNKPTD DIVLMAQTLE KIFLQKVASM PQEEQELVVT IPKNSHKKGA
210 220 230 240 250
KLAALQGSVT SAHQVPAVSS VSHTALYTPP PEIPTTVLNI PHPSVISSPL
260 270 280 290 300
LKSLHSAGPP LLAVTAAPPA QPLAKKKGVK RKADTTTPTP TAILAPGSPA
310 320 330 340 350
SPPGSLEPKA ARLPPMRRES GRPIKPPRKD LPDSQQQHQS SKKGKLSEQL
360 370 380 390 400
KHCNGILKEL LSKKHAAYAW PFYKPVDASA LGLHDYHDII KHPMDLSTVK
410 420 430 440 450

RKMENRDYRD AQEFAADVRL MFSNCYKYNP PDHDVVAMAR KLQDVFEFRY
460 470 480 490 500
AKMPDEPLEP GPLPVSTAMP PGLAKSSSES SSEESSSESS SEEEEEEDEE
510 520 530 540 550
DEEEEESESS DSEEERAHRL AELQEQLRAV HEQLAALSQG PISKPKRKRE
560 570 580 590 600
KKEKKKKRKA EKHRGRAGAD EDDKGPRAPR PPQPKKSKKA SGSGGGSAAL
610 620 630 640 650
GPSGFGPSGG SGTKLPKKAT KTAPPALPTG YDSEEEEESR PMSYDEKRQL
660 670 680 690 700
SLDINKLPGE KLGRVVHIIQ AREPSLRDSN PEEIEIDFET LKPSTLRELE
710 720 730 740 750
RYVLSCLRKK PRKPYTIKKP VGKTKEELAL EKKRELEKRL QDVSGQLNST
760 770 780 790 800
KKPPKKANEK TESSSAQQVA VSRLSASSSS SDSSSSSSSS SSSDTSDSDS

In one aspect, the terms "co-administered" and "co-administration" as relating to a subject refer to administering to the subject a compound of the disclosure, or a derivative, solvate, salt or prodrug salt thereof, along with a compound and/or composition that may also treat the disorders or diseases contemplated within the disclosure. In certain embodiments, the co-administered compounds and/or compositions are administered separately, or in any kind of combination as part of a single therapeutic approach. The co-administered compound and/or composition may be formulated in any kind of combinations as mixtures of solids and liquids under a variety of solid, gel, and liquid formulations, and as a solution.

As used herein, "differentially expressed" or "differential expression" refers to any statistically significant difference (p<0.05) in the level of expression of a gene between two samples (e.g., two biological samples), or between a sample and a reference standard. Whether a difference in expression between two samples is statistically significant can be determined using an appropriate t-test (*e.g.,* one- sample t-test, two-sample t-test, Welch's t-test) or other statistical test known to those of skill in the art.

A "disease" as used herein is a state of health of an animal wherein the animal cannot maintain homeostasis, and wherein if the disease is not ameliorated then the animal's health continues to deteriorate.

A "disorder" as used herein in an animal is a state of health in which the animal is able to maintain homeostasis, but in which the animal's state of health is less favorable than it would be in the absence of the disorder. Left untreated, a disorder does not necessarily cause a further decrease in the animal's state of health.

As used herein, the term "down-regulation" or "downregulation" as relating to a protein, ligand and/or receptor refers to any process or mechanism that reduces, or slows or prevents the increase of, the concentration, expression level and/or activity of the protein, ligand and/or receptor. The term "down-regulation" or "downregulation" includes any process or mechanism described herein or known to those skilled in the art, including *in vivo* or *ex vivo* methods.

The terms "dsRNAi", "RNAi", "iRNA", and "siRNA" are used interchangeably herein unless otherwise noted.

As used herein, the terms "effective amount" or "therapeutically effective amount" or "pharmaceutically effective amount" of a compound are used interchangeably to refer to the amount of the compound and/or composition which is sufficient to provide a beneficial effect to the subject to which the compound and/or composition is administered. The term to "treat," as used herein, means reducing the frequency with which symptoms are experienced by a patient or subject or administering an agent or compound to reduce the severity with which symptoms are experienced. An appropriate therapeutic amount in any individual case may be determined by one of ordinary skill in the art using routine experimentation.

As used herein, "encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (*i.e.,* rRNA, tRNA and mRNA) or a defined sequence of amino acids and the biological properties resulting therefrom. Thus, a gene encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, may be referred to as encoding the protein or other product of that gene or cDNA.

As used herein, the terms "gene expression" and "expression of the gene" are interchangeable. Both refer to the translation of information encoded in a gene into a gene product (*e.g.,* RNA, protein). Expressed genes include genes that are transcribed into RNA (*e.g.,* mRNA) that is subsequently translated into protein, as well as genes that are transcribed into non-coding functional RNA molecules that are not translated into protein (*e.g*., transfer RNA (tRNA), ribosomal RNA (rRNA), microRNA, ribozymes). "Level of expression," "expression level" or "expression intensity" refers to the level (e.g., amount) of one or more products (*e.g*., mRNA, protein) encoded by a given gene in a sample or reference standard.

As used herein, the term "HDAC11" (SEQ ID NO:2) refers to a 39kDa histone deacetylase enzyme that in humans is encoded by the *HDAC11* gene on chromosome 3 in humans. HDAC11 is the only Class IV HDAC, and is the smallest HDAC isoform, with the following sequence (an isoform has amino acids 1-28 and 85-101 missing).

10 20 30 40 50
MLHTTQLYQH VPETRWPIVY SPRYNITFMG LEKLHPFDAG KWGKVINFLK
60 70 80 90 100
EEKLLSDSML VEAREASEED LLVVHTRRYL NELKWSFAVA TITEIPPVIF
110 120 130 140 150
LPNFLVQRKV LRPLRTQTGG TIMAGKLAVE RGWAINVGGG FHHCSSDRGG
160 170 180 190 200
GFCAYADITL AIKFLFERVE GISRATIIDL DAHQGNGHER DFMDDKRVYI
210 220 230 240 250
MDVYNRHIYP GDRFAKQAIR RKVELEWGTE DDEYLDKVER NIKKSLQEHL
260 270 280 290 300
PDVVVYNAGT DILEGDRLGG LSISPAGIVK RDELVFRMVR GRRVPILMVT
310 320 330 340
SGGYQKRTAR IIADSILNLF GLGLIGPESP SVSAQNSDTP LLPPAVP

As used herein, the term "immunoglobulin" or "Ig" is defined as a class of proteins that function as antibodies. The five members included in this class of proteins are IgA, IgG, IgM, IgD, and IgE. IgA is the primary antibody that is present in body secretions, such as saliva, tears, breast milk, gastrointestinal secretions and mucus secretions of the respiratory and genitor-urinary tracts. IgG is the most common circulating antibody. IgM is the main immunoglobulin produced in the primary immune response in most mammals. It is the most efficient immunoglobulin in agglutination, complement fixation, and other antibody responses, and is important in defense against bacteria and viruses. IgD is the immunoglobulin that has no known antibody function, but may serve as an antigen receptor. IgE is the immunoglobulin that mediates immediate hypersensitivity by causing release of mediators from mast cells and basophils upon exposure to allergen.

The terms "inhibit" and "antagonize", as used herein, mean to reduce a molecule, a reaction, an interaction, a gene, an mRNA, and/or a protein's expression, stability, function or activity by a measurable amount or to prevent entirely. Inhibitors are compounds that, *e.g.,* bind to, partially or totally block stimulation, decrease, prevent, delay activation, inactivate, desensitize, or down regulate a protein, a gene, and an mRNA stability, expression, function and activity, *e.g.,* antagonists.

"Instructional material," as that term is used herein, includes a publication, a recording, a diagram, or any other medium of expression which can be used to communicate the usefulness of the composition and/or compound of the disclosure in a kit. The instructional material of the kit may, for example, be affixed to a container that contains the compound and/or composition of the disclosure or be shipped together with a container which contains the compound and/or composition. Alternatively, the instructional material may be shipped separately from the container with the intention that the recipient uses the instructional material and the compound cooperatively. Delivery of the instructional material may be, for example, by physical delivery of the publication or other medium of expression communicating the usefulness of the kit, or may alternatively be achieved by electronic transmission, for example by means of a computer, such as by electronic mail, or download from a website.

The terms "patient," "subject" or "individual" are used interchangeably herein, and refer to any animal, or cells thereof whether *in vitro* or *in situ,* amenable to the methods described herein. In a non-limiting embodiment, the patient, subject or individual is a human.

The term "overexpression" refers to a gene producing more gene products (e.g. mRNA, protein) in relative to a control.

"Parenteral" administration of a composition includes, *e.g.,* subcutaneous (s.c.), intravenous (i.v.), intramuscular (i.m.), or intrasternal injection, or infusion techniques.

As used herein, the term "pharmaceutical composition" refers to a mixture of at least one compound and/or composition useful within the disclosure with other chemical components, such as carriers, stabilizers, diluents, dispersing agents, suspending agents, thickening agents, and/or excipients. The pharmaceutical composition facilitates administration of the compound and/or composition to an organism. Multiple techniques of administering a compound and/or composition exist in the art including, but not limited to: intravenous, oral, aerosol, parenteral, ophthalmic, pulmonary, intracranial and topical administration.

As used herein, the term "pharmaceutically acceptable" refers to a material, such as a carrier or diluent, which does not abrogate the biological activity or properties of the composition, and is relatively non-toxic, *i.e.,* the material may be administered to an individual without causing undesirable biological effects or interacting in a deleterious manner with any of the components of the composition in which it is contained.

As used herein, the term "pharmaceutically acceptable carrier" means a pharmaceutically acceptable material, composition or carrier, such as a liquid or solid filler, stabilizer, dispersing agent, suspending agent, diluent, excipient, thickening agent, solvent or encapsulating material, involved in carrying or transporting a compound useful within the disclosure within or to the patient such that it may perform its intended function. Typically, such constructs are carried or transported from one organ, or portion of the body, to another organ, or portion of the body. Each carrier must be "acceptable" in the sense of being compatible with the other ingredients of the formulation, including the compound useful within the disclosure, and not injurious to the patient. Some examples of materials that may serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; surface active agents; alginic acid; pyrogen-free water; isotonic saline; Ringer's solution; ethyl alcohol; phosphate buffer solutions; and other non-toxic compatible substances employed in pharmaceutical formulations. As used herein, "pharmaceutically acceptable carrier" also includes any and all coatings, antibacterial and antifungal agents, and absorption delaying agents, and the like that are compatible with the activity of the compound useful within the disclosure, and are physiologically acceptable to the patient. Supplementary active compounds may also be incorporated into the compositions. The "pharmaceutically acceptable carrier" may further include a pharmaceutically acceptable salt, prodrug, solvate or derivative of the compound useful within the disclosure. Other additional ingredients that may be included in the pharmaceutical compositions used in the practice of the disclosure are known in the art and described, for example in Remington's Pharmaceutical Sciences (Genaro, Ed., Mack Publishing Co., 1985, Easton, PA).

As used herein, the language "pharmaceutically acceptable salt" refers to a salt of the administered compounds prepared from pharmaceutically acceptable non-toxic acids, including inorganic acids, organic acids, solvates, hydrates, or clathrates thereof.

The term "prevent," "preventing" or "prevention," as used herein, means avoiding or delaying the onset of symptoms associated with a disease or condition in a subject that has not developed such symptoms at the time the administering of an agent or compound commences.

"Primer" refers to a polynucleotide that is capable of specifically hybridizing to a designated polynucleotide template and providing a point of initiation for synthesis of a complementary polynucleotide. Such synthesis occurs when the polynucleotide primer is placed under conditions in which synthesis is induced, *i.e.,* in the presence of nucleotides, a complementary polynucleotide template, and an agent for polymerization such as DNA polymerase. A primer is typically single-stranded, but may be double-stranded. Primers are typically deoxyribonucleic acids, but a wide variety of synthetic and naturally occurring primers are useful for many applications. A primer is complementary to the template to which it is designed to hybridize to serve as a site for the initiation of synthesis, but need not reflect the exact sequence of the template. In such a case, specific hybridization of the primer to the template depends on the stringency of the hybridization conditions. Primers may be labeled with, e.g., chromogenic, radioactive, or fluorescent moieties and used as detectable moieties.

"Probe" refers to a polynucleotide that is capable of specifically hybridizing to a designated sequence of another polynucleotide. A probe specifically hybridizes to a target complementary polynucleotide, but need not reflect the exact complementary sequence of the template. In such a case, specific hybridization of the probe to the target depends on the stringency of the hybridization conditions. Probes may be labeled with, *e.g.,* chromogenic, radioactive, or fluorescent moieties and used as detectable moieties.

The term "promoter" as used herein is defined as a DNA sequence recognized by the synthetic machinery of the cell, or introduced synthetic machinery, required to initiate the specific transcription of a polynucleotide sequence.

As used herein, the term "promoter/regulatory sequence" means a nucleic acid sequence which is required for expression of a gene product operably linked to the promoter/ regulatory sequence. In some instances, this sequence may be the core promoter sequence and in other instances, this sequence may also include an enhancer sequence and other regulatory elements that are required for expression of the gene product. The promoter/ regulatory sequence may for example be one that expresses the gene product in a tissue specific manner.

The term "recombinant DNA" as used herein is defined as DNA produced by joining pieces of DNA from different sources.

The term "recombinant polypeptide" as used herein is defined as a polypeptide produced by using recombinant DNA methods.

The term "RNA" as used herein is defined as ribonucleic acid.

By the term "specifically bind" or "specifically binds," as used herein, is meant that a first molecule (*e.g.,* an antibody) preferentially binds to a second molecule (*e.g.,* a particular antigenic epitope), but does not necessarily bind only to that second molecule.

As used herein, a "subject" refers to a human or non-human mammal. Non-human mammals include, for example, livestock and pets, such as ovine, bovine, porcine, canine, feline and murine mammals. In certain embodiments, the subject is human.

By the term "synthetic antibody" as used herein, is meant an antibody which is generated using recombinant DNA technology, such as, for example, an antibody expressed by a bacteriophage as described herein. The term should also be construed to mean an antibody which has been generated by the synthesis of a DNA molecule encoding the antibody and which DNA molecule expresses an antibody protein, or an amino acid sequence specifying the antibody, wherein the DNA or amino acid sequence has been obtained using synthetic DNA or amino acid sequence technology which is available and well known in the art.

The term "transfected" or "transformed" or "transduced" as used herein refers to a process by which exogenous nucleic acid is transferred or introduced into the host cell. A "transfected" or "transformed" or "transduced" cell is one that has been transfected, transformed or transduced with exogenous nucleic acid. The cell includes the primary subject cell and its progeny.

As used herein, the term "treatment" or "treating" is defined as the application or administration of a therapeutic agent, *i.e.,* a composition useful within the disclosure (alone or in combination with another pharmaceutical agent), to a subject, or application or administration of a therapeutic agent to an isolated tissue or cell line from a subject (*e.g.,* for diagnosis or *ex vivo* applications), who has a disease or disorder, a symptom of a disease or disorder or the potential to develop a disease or disorder, with the purpose to cure, heal, alleviate, relieve, alter, remedy, ameliorate, improve or affect the disease or disorder, the symptoms of the disease or disorder or the potential to develop the disease or disorder. Such treatments may be specifically tailored or modified, based on knowledge obtained from the field of pharmacogenomics.

The term "underexpression" refers to a gene that does not produce or produce fewer gene products (*e.g.* mRNA, protein) in relative to a control.

The following abbreviations are used herein: BAT, brown adipose tissue; HDL, high-density lipoprotein; eWAT, epididymal white adipose tissue; HDAC11, histone deacetylase 11; iBAT, interscapular brown adipose tissue; ingWAT, inguinal white adipose tissue; KO, knockout; MEF, mouse embryonic fibroblast; MR, metabolic rate; RER, respiratory exchange ratio; WAT, white adipose tissue; and WT, wild-type.

Throughout this disclosure, various aspects of the disclosure can be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

### Compositions

The inhibition of a targeted gene expression (*e.g., HDAC11*) is well known in the art. It can be achieved using, for example, DNA binding agents, small molecules, siRNAs, antibodies, antisense RNAs, modified antisense RNAs (such as morpholino oligomers), synthetic oligonucleotides or any combinations thereof. It may be also achieved post-transcriptionally through RNA interference. Alternatively, one may inhibit the activity of HDAC11 itself.

According to the present disclosure, the composition comprises an agent selected from the group consisting of an antibody, siRNA, antisense RNA, modified antisense RNA, a small molecule inhibitor and any combinations thereof. In other embodiments, the antibody comprises an antibody selected from a polyclonal antibody, monoclonal antibody, humanized antibody, synthetic antibody, heavy chain antibody, human antibody, biologically active fragment of an antibody, and combinations thereof.

In certain embodiments, the inhibitor comprises an isolated nucleic acid. In other embodiments, the inhibitor is an siRNA or antisense molecule, which inhibits HDAC11 expression. In yet other embodiments, the nucleic acid comprises a promoter/regulatory sequence such that the nucleic acid is preferably capable of directing expression of the nucleic acid. Thus, the disclosure provides expression vectors and methods for the introduction of exogenous DNA into cells with concomitant expression of the exogenous DNA in the cells such as those described, for example, in Sambrook et al. (2012, Molecular Cloning: A Laboratory Manual, Cold Spring Harbor Laboratory, New York), and in Ausubel et al. (1997, Current Protocols in Molecular Biology, John Wiley & Sons, New York) and as described elsewhere herein.

In certain embodiments, the subject is a mammal. In other embodiments, the mammal is human. In yet other embodiments, the composition is administered by an inhalational, oral, rectal, vaginal, parenteral, intracranial, topical, transdermal, pulmonary, intranasal, buccal, ophthalmic, intrathecal, or intravenous route of administration.

Methods for identifying an agent that inhibits HDAC11 expression and/or activity, or induces HDAC11 degradation, are known in the art, or provided herein. In a non-limiting embodiment, recombinant HDAC11 can be overexpressed in a pre-adipocyte and/or adipocyte cell (*in vivo* and/or *in vitro*)*,* thus leading to overall reduction of brown fat levels in that cell. Such modified cell can be used, in a non-limiting example, for identifying test compounds that are useful within the present methods. If contacting the modified cell with the test compound causes an increase, prevents further decrease, and/or reduces the rate of decrease, of brown fat levels in that cell, the compound is identified as being able to inhibit HDAC11 expression and/or activity, or induce HDAC11 degradation, in the cell.

According to the present disclosure, the agent inhibits HDAC11 expression and/or activity, or induces HDAC11 degradation. In other embodiments, the agent is selective in inhibiting HDAC11 expression and/or activity, or inducing HDAC11 degradation. In yet other embodiments, the agent is selective in inhibiting HDAC11 expression and/or activity, or inducing HDAC11 degradation, over other HDACs, such as but not limited to, HDAC1, HDAC2, HDAC3, HDAC4, HDAC5, HDAC6, HDAC7, HDAC8, HDAC9, and/or HDAC10. In yet other embodiments, the selectivity is equal to, or greater than, a factor of about 3, about 10, about 30, about 100, about 300, about 1,000, about 3,000, about 10,000, about 30,000, about 100,000, about 300,000, and/or about 1,000,000, or any multiples thereof.

Synthetic nucleic acids are one of the ways to inhibit a targeted gene expression (e.g. *HDAC11*)*.* Non-limiting representatives of these synthetic nucleic acids (oligonucleotides) are antisense oligonucleotides, ribozymes, DNA enzymes and external guide sequences (EGS).

Antisense molecules and their use for inhibiting gene expression are well known in the art (*see, e.g.,* Cohen, 1989, In: Oligodeoxyribonucleotides, Antisense Inhibitors of Gene Expression, CRC Press). "Antisense oligonucleotides" are short single-stranded nucleic acid derivatives that bind to a complementary messenger ribonucleic acid (mRNA) which translation into the corresponding protein is to be inhibited. In the cell, antisense nucleic acids hybridize to the corresponding mRNA, forming a double-stranded molecule thereby inhibiting the translation of genes. The use of antisense methods to inhibit the translation of genes is known in the art, and is described, for example, in Marcus-Sakura, 1988, Anal. Biochem. 172:289. Such antisense molecules may be provided to the cell via genetic expression using DNA encoding the antisense molecule as taught by U.S. Patent No. 5,190,931. Alternatively, antisense molecules of the disclosure may be made synthetically and then provided to the cell. Antisense oligomers of between about 10 to about 30, and more preferably about 15 nucleotides, are preferred, since they are easily synthesized and introduced into a target cell. Synthetic antisense molecules contemplated by the disclosure include oligonucleotide derivatives known in the art which have improved biological activity compared to unmodified oligonucleotides (U.S. Patent No. 5,023,243).

In certain embodiments, an antisense nucleic acid sequence expressed by a plasmid vector is used to inhibit HDAC11 protein expression. The antisense expressing vector is used to transfect a mammalian cell or the mammal itself, thereby causing reduced endogenous expression of HDAC11.

The ability to specifically inhibit gene function in a variety of organisms utilizing antisense RNA or dsRNA-mediated interference (RNAi or dsRNA) is well known in the fields of molecular biology.

RNA interference (RNAi) is a phenomenon in which the introduction of double-stranded RNA (dsRNA) into a diverse range of organisms and cell types causes degradation of the complementary mRNA. In the cell, long dsRNAs are cleaved into short 21-25 nucleotide small interfering RNAs, or siRNAs, by a ribonuclease known as Dicer. The siRNAs subsequently assemble with protein components into an RNA-induced silencing complex (RISC), unwinding in the process. Activated RISC then binds to complementary transcript by base pairing interactions between the siRNA antisense strand and the mRNA. The bound mRNA is cleaved and sequence specific degradation of mRNA results in gene silencing. See, for example, U.S. Patent No. 6,506,559; Fire et al., 1998, Nature 391(19):306-311; Timmons et al., 1998, Nature 395:854; Montgomery et al., 1998, TIG 14 (7):255-258; Engelke, Ed., RNA Interference (RNAi) Nuts & Bolts of RNAi Technology, DNA Press, Eagleville, PA (2003); and Hannon, Ed., RNAi A Guide to Gene Silencing, Cold Spring Harbor Laboratory Press, Cold Spring Harbor, NY (2003). Soutschek et al. (2004, Nature 432:173-178) describes a chemical modification to siRNAs that aids in intravenous systemic delivery. Optimizing siRNAs involves consideration of overall G/C content, C/T content at the termini, Tm and the nucleotide content of the 3' overhang. See, for instance, Schwartz et al., 2003, Cell, 115:199-208 and Khvorova et al., 2003, Cell 115:209-216. Therefore, the present disclosure also includes methods of decreasing levels of HDAC11 using RNAi technology.

dsRNA (RNAi) typically comprises a polynucleotide sequence identical or homologous to a target gene (or fragment thereof) linked directly, or indirectly, to a polynucleotide sequence complementary to the sequence of the target gene (or fragment thereof). The dsRNA may comprise a polynucleotide linker sequence of sufficient length to allow for the two polynucleotide sequences to fold over and hybridize to each other; however, a linker sequence is not necessary. The linker sequence is designed to separate the antisense and sense strands of RNAi significantly enough to limit the effects of steric hindrances and allow for the formation of dsRNA molecules and should not hybridize with sequences within the hybridizing portions of the dsRNA molecule. The specificity of this gene silencing mechanism appears to be extremely high, blocking expression only of targeted genes, while leaving other genes unaffected. Accordingly, one method for treating retroviral infection according to the disclosure comprises the use of materials and methods utilizing double-stranded interfering RNA (dsRNAi), or RNA-mediated interference (RNAi) comprising polynucleotide sequences identical or homologous to a desired component of TGF-β signaling pathway. A simple injection of dsRNA, whose sense-strand sequence is identical to the target mRNA to be inhibited, can specifically inhibit expression of a target gene having the corresponding DNA sequence. This does not impair the expression of nonhomologous genes and the base sequence of the target gene is not altered.

RNA containing a nucleotide sequence identical to a fragment of the target gene is preferred for inhibition; however, RNA sequences with insertions, deletions, and point mutations relative to the target sequence may also be used for inhibition. Sequence identity may optimized by sequence comparison and alignment algorithms known in the art (Gribskov & Devereux, Sequence Analysis Primer, Stockton Press, 1991, and references cited therein) and calculating the percent difference between the nucleotide sequences by, for example, the Smith-Waterman algorithm as implemented in the BESTFIT software program using default parameters (e.g., University of Wisconsin Genetic Computing Group). Alternatively, the duplex region of the RNA may be defined functionally as a nucleotide sequence that is capable of hybridizing with a fragment of the target gene transcript.

RNA may be synthesized either *in vivo* or *in vitro.* Endogenous RNA polymerase of the cell may mediate transcription *in vivo,* or cloned RNA polymerase may be used for transcription *in vivo* or *in vitro.* For transcription from a transgene *in vivo* or an expression construct, a regulatory region (e.g., promoter, enhancer, silencer, splice donor and acceptor, polyadenylation) may be used to transcribe the RNA strand (or strands); the promoters may be known inducible promoters such as baculovirus. Inhibition may be targeted by specific transcription in an organ, tissue, or cell type. The RNA strands may or may not be polyadenylated; the RNA strands may or may not be capable of being translated into a polypeptide by a cell's translational apparatus. RNA may be chemically or enzymatically synthesized by manual or automated reactions. The RNA may be synthesized by a cellular RNA polymerase or bacteriophage RNA polymerase (*e.g.,* T3, T7, SP6). The use and production of an expression construct are known in the art (for example, WO 97/32016; U.S. Patent Nos. 5,593,874; 5,698,425; 5,712,135; 5,789,214; and 5,804,693; and references cited therein). If synthesized chemically or by *in vitro* enzymatic synthesis, the RNA may be purified prior to introduction into the cell. For example, RNA may be purified from a mixture by extraction with a solvent or resin, precipitation, electrophoresis, chromatography, or combinations thereof. Alternatively, the RNA may be used with no, or a minimum of, purification to avoid losses due to sample processing. The RNA may be dried for storage or dissolved in an aqueous solution. The solution may contain buffers or salts to promote annealing and/or stabilization of duplex strands.

Fragments of genes may also be utilized for targeted suppression of gene expression. These fragments are typically in the approximate size range of about 20 consecutive nucleotides of a target sequence. Thus, targeted fragments are preferably at least about 15 consecutive nucleotides. In certain embodiments, the gene fragment targeted by the RNAi molecule is about 20-25 consecutive nucleotides in length. In a more preferred embodiment, the gene fragments are at least about 25 consecutive nucleotides in length. In an even more preferred embodiment, the gene fragments are at least 50 consecutive nucleotides in length. Various embodiments also allow for the joining of one or more gene fragments of at least about 15 nucleotides via linkers. Thus, RNAi molecules useful in the practice of the instant disclosure may contain any number of gene fragments joined by linker sequences.

In certain embodiments, the disclosure provides a vector comprising an siRNA or antisense polynucleotide. In other embodiments, the siRNA or antisense polynucleotide inhibits the expression of HDAC11. The incorporation of a desired polynucleotide into a vector and the choice of vectors is well-known in the art.

In certain embodiments, the expression vectors described herein encode a short hairpin RNA (shRNA) inhibitor. shRNA inhibitors are well known in the art and are directed against the mRNA of a target, thereby decreasing the expression of the target. In certain embodiments, the encoded shRNA is expressed by a cell, and is then processed into siRNA. For example, in certain instances, the cell possesses native enzymes (e.g., dicer) that cleaves the shRNA to form siRNA.

The siRNA, shRNA, or antisense polynucleotide can be cloned into a number of types of vectors as described elsewhere herein. For expression of the siRNA or antisense polynucleotide, at least one module in each promoter functions to position the start site for RNA synthesis.

In order to assess the expression of the siRNA, shRNA, or antisense polynucleotide, the expression vector to be introduced into a cell can also contain either a selectable marker gene or a reporter gene or both to facilitate identification and selection of expressing cells from the population of cells sought to be transfected or infected using a viral vector. In certain embodiments, the selectable marker may be carried on a separate piece of DNA and used in a co-transfection procedure. Both selectable markers and reporter genes may be flanked with appropriate regulatory sequences to enable expression in the host cells. Useful selectable markers are known in the art and include, for example, antibiotic-resistance genes, such as neomycin resistance and the like.

Therefore, in another aspect, the disclosure relates to a vector, comprising the nucleotide sequence of the disclosure or the construct of the disclosure. The choice of the vector will depend on the host cell in which it is to be subsequently introduced. In certain embodiments, the vector of the disclosure is an expression vector. Suitable host cells include a wide variety of prokaryotic and eukaryotic host cells. In certain embodiments, the expression vector is selected from the group consisting of a viral vector, a bacterial vector and a mammalian cell vector. Prokaryote- and/or eukaryote-vector based systems can be employed for use with the present disclosure to produce polynucleotides, or their cognate polypeptides. Many such systems are commercially and widely available.

Further, the expression vector may be provided to a cell in the form of a viral vector. Viral vector technology is well known in the art and is described, for example, in virology and molecular biology manuals. Viruses, which are useful as vectors include, but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers. See, *e.g.,* WO 01/96584; WO 01/29058; and U.S. Pat. No. 6,326,193.

By way of illustration, the vector in which the nucleic acid sequence is introduced can be a plasmid that is or is not integrated in the genome of a host cell when it is introduced in the cell. Illustrative, non-limiting examples of vectors in which the nucleotide sequence of the disclosure or the gene construct of the disclosure can be inserted include a tet-on inducible vector for expression in eukaryote cells.

The vector may be obtained by conventional methods known by persons skilled in the art. In certain embodiments, the vector is a vector useful for transforming animal cells.

In certain embodiments, the recombinant expression vectors may also contain nucleic acid molecules which encode a peptide or peptidomimetic inhibitor of disclosure, described elsewhere herein.

A promoter may be one naturally associated with a gene or polynucleotide sequence, as may be obtained by isolating the 5' non-coding sequences located upstream of the coding segment and/or exon. Such a promoter can be referred to as "endogenous." Similarly, an enhancer may be one naturally associated with a polynucleotide sequence, located either downstream or upstream of that sequence. Alternatively, certain advantages will be gained by positioning the coding polynucleotide segment under the control of a recombinant or heterologous promoter, which refers to a promoter that is not normally associated with a polynucleotide sequence in its natural environment. A recombinant or heterologous enhancer refers also to an enhancer not normally associated with a polynucleotide sequence in its natural environment. Such promoters or enhancers may include promoters or enhancers of other genes, and promoters or enhancers isolated from any other prokaryotic, viral, or eukaryotic cell, and promoters or enhancers not "naturally occurring," *i.e*., containing different elements of different transcriptional regulatory regions, and/or mutations that alter expression. In addition to producing nucleic acid sequences of promoters and enhancers synthetically, sequences may be produced using recombinant cloning and/or nucleic acid amplification technology, including PCR^{™}, in connection with the compositions disclosed herein (U.S. Patent 4,683,202, U.S. Patent 5,928,906). Furthermore, it is contemplated the control sequences that direct transcription and/or expression of sequences within non-nuclear organelles such as mitochondria, chloroplasts, and the like, can be employed as well.

It will be important to employ a promoter and/or enhancer that effectively directs the expression of the DNA segment in the cell type, organelle, and organism chosen for expression. Those of skill in the art of molecular biology generally know how to use promoters, enhancers, and cell type combinations for protein expression. The promoters employed may be constitutive, tissue-specific, inducible, and/or useful under the appropriate conditions to direct high level expression of the introduced DNA segment, such as is advantageous in the large-scale production of recombinant proteins and/or peptides. The promoter may be heterologous or endogenous.

The recombinant expression vectors may also contain a selectable marker gene which facilitates the selection of transformed or transfected host cells. Suitable selectable marker genes are genes encoding proteins such as G418 and hygromycin which confer resistance to certain drugs, β-galactosidase, chloramphenicol acetyltransferase, firefly luciferase, or an immunoglobulin or portion thereof such as the Fc portion of an immunoglobulin preferably IgG. The selectable markers may be introduced on a separate vector from the nucleic acid of interest.

Following the generation of the siRNA polynucleotide, a skilled artisan will understand that the siRNA polynucleotide has certain characteristics that can be modified to improve the siRNA as a therapeutic compound. Therefore, the siRNA polynucleotide may be further designed to resist degradation by modifying it to include phosphorothioate, or other linkages, methylphosphonate, sulfone, sulfate, ketyl, phosphorodithioate, phosphoramidate, phosphate esters, and the like (see, *e.g.,* Agrwal et al., 1987, Tetrahedron Lett. 28:3539-3542; Stec et al., 1985 Tetrahedron Lett. 26:2191-2194; Moody et al., 1989 Nucleic Acids Res. 12:4769-4782; Eckstein, 1989 Trends Biol. Sci. 14:97-100; Stein, In: Oligodeoxynucleotides. Antisense Inhibitors of Gene Expression, Cohen, ed., Macmillan Press, London, pp. 97-117 (1989)).

Any polynucleotide may be further modified to increase its stability *in vivo.* Possible modifications include, but are not limited to, the addition of flanking sequences at the 5' and/or 3' ends; the use of phosphorothioate or 2' O-methyl rather than phosphodiester linkages in the backbone; and/or the inclusion of nontraditional bases such as inosine, queosine, and wybutosine and the like, as well as acetyl- methyl-, thio- and other modified forms of adenine, cytidine, guanine, thymine, and uridine.

In certain embodiments, inhibiting the activity of HDAC11 can be accomplished by using a transdominant negative mutant.

Ribozymes and their use for inhibiting gene expression are also well known in the art (*see, e.g.,* Cech et al., 1992, J Biol Chem 267:17479-17482; Hampel et al., 1989, Biochemistry 28:4929-4933; Eckstein et al., WO 92/07065; U.S. Patent No. 5,168,053). Ribozymes are RNA molecules possessing the ability to specifically cleave other single-stranded RNA in a manner analogous to DNA restriction endonucleases. Through the modification of nucleotide sequences encoding these RNAs, molecules may be engineered to recognize specific nucleotide sequences in an RNA molecule and cleave it (Cech, 1988, J. Amer. Med. Assn. 260:3030). A major advantage of this approach is the fact that ribozymes are sequence-specific.

There are two basic types of ribozymes, namely, tetrahymena-type (Hasselhoff, 1988, Nature 334:585) and hammerhead-type. Tetrahymena-type ribozymes recognize sequences that are four bases in length, while hammerhead-type ribozymes recognize base sequences 11-18 bases in length. The longer the sequence, the greater the likelihood that the sequence will occur exclusively in the target mRNA species. Consequently, hammerhead-type ribozymes are preferable to tetrahymena-type ribozymes for inactivating specific mRNA species, and 18-base recognition sequences are preferable to shorter recognition sequences which may occur randomly within various unrelated mRNA molecules.

Ribozymes useful for inhibiting the expression of HDAC11 may be designed by incorporating target sequences into the basic ribozyme structure that are complementary to the mRNA sequence of HDAC11. Ribozymes targeting HDAC11 may be synthesized using commercially available reagents (Applied Biosystems, Inc., Foster City, CA) or they may be genetically expressed from DNA encoding them.

EGS are synthetic RNA analogs which activate the cellular RNase P and bind via appropriate flanking sequences to the target mRNA and induce a specific mRNA degradation.

It is also possible to inhibit HDAC11 gene expression by interaction with particular proteins with the aid of "decoy" oligomers, which mimic the binding regions for transcription factors. Treatment with decoy agents makes it possible to intercept particular proteins, in particular transcription factors, in a sequence-specific manner and thereby prevent a transcription activation.

Inhibition of HDAC11 can be achieved by agents such as small molecules, antibodies, peptides and enzymes. As will be understood by one skilled in the art, any antibody that may recognize and specifically bind to HDAC11 is useful in the present disclosure. The disclosure should not be construed to be limited to any one type of antibody, either known or heretofore unknown, provided that the antibody may specifically bind to HDAC11. Methods of making and using such antibodies are well known in the art. For example, the generation of polyclonal antibodies may be accomplished by inoculating the desired animal with the antigen and isolating antibodies which specifically bind the antigen therefrom. Monoclonal antibodies directed against full length or peptide fragments of a protein or peptide may be prepared using any well-known monoclonal antibody preparation procedures, such as those described, for example, in Harlow et al. (1989, Antibodies, A Laboratory Manual, Cold Spring Harbor, New York) and in Tuszynski et al. (1988, Blood 72:109-115). Quantities of the desired peptide may also be synthesized using chemical synthesis technology. Alternatively, DNA encoding the desired peptide may be cloned and expressed from an appropriate promoter sequence in cells suitable for the generation of large quantities of peptide. Monoclonal antibodies directed against the peptide are generated from mice immunized with the peptide using standard procedures as referenced herein. However, the disclosure should not be construed as being limited solely to methods and compositions including these antibodies, but should be construed to include other antibodies, as that term is defined elsewhere herein.

Gene expression can be evaluated in a biological sample from a subject. A biological sample can be a tissue sample, a biological fluid sample, a cell (*e.g.,* a tumor cell) sample, and the like. Any means of sampling from a subject, for example, tissue biopsy, by blood draw, spinal tap, tissue smear or scrape can be used to obtain a biological sample. Thus, the biological sample can be a biopsy specimen (*e.g.*, tumor, polyp, mass (solid, cell)), aspirate, smear or blood sample. A tumor sample can also be obtained by *in vitro* harvest of cultured human cells derived from an individual's tissue. Samples can, if desired, be stored before analysis by suitable storage means that preserve a sample's protein and/or nucleic acid in an analyzable condition, such as quick freezing, or a controlled freezing regime.

The presence or absence of gene expression can be ascertained by the methods described herein or other suitable assays known to those of skill in the art.

A difference (*e.g.,* an increase, a decrease) in *HDAC11* gene expression can be determined by comparison of the level of expression of the gene in a biological sample from a subject to that of a suitable control. The reference standard can be a typical, normal or normalized range of levels, or a particular level, of expression of a protein or RNA (*e.g.,* an expression standard). The standards can comprise, for example, a zero gene expression level, the gene expression level in a standard cell line, or the average level of gene expression previously obtained for a population of normal human controls. Thus, the method does not necessarily require that expression of the gene be assessed in, or compared to, a control sample. A determination of difference (*e.g.,* an increase, a decrease) in the level of expression of a gene between two samples, or between a sample and a reference standard, can be accomplished using an appropriate algorithm or statistical analysis, several of which are known to those of skill in the art.

Suitable assays that can be used to assess the level of expression of a gene in a biological sample from a subject are known to those of skill in the art. For example, the level of an RNA (*e.g.,* mRNA) gene product in a sample can be determined by any technique that is suitable for detecting RNA expression levels in a biological sample. Several suitable techniques for determining RNA expression levels in cells from a biological sample (*e.g.,* Northern blot analysis, RT-PCR, in situ hybridization) are well known to those of skill in the art. Detection and quantification of specific RNA is accomplished using appropriately labeled DNA or RNA probes complementary to the RNA in question. See, for example, Molecular Cloning: A Laboratory Manual, J. Sambrook et al., eds., 2nd edition, Cold Spring Harbor Laboratory Press, 1989, Chapter 7. Other techniques for measuring gene expression in a sample are also within the skill in the art, and include various techniques for measuring rates of RNA transcription and degradation.

Gene expression on an array or gene chip can be assessed using an appropriate algorithm (*e.g.,* statistical algorithm). Suitable software applications for assessing gene expression levels using a microarray or gene chip are known in the art.

Methods for detecting HDAC11 include, for example, immunological and immunochemical methods, such as flow cytometry (*e.g.,* FACS analysis), enzyme-linked immunosorbent assays (ELISA), chemiluminescence assays, radioimmunoassay, immunoblot (*e.g.,* Western blot), immunohistochemistry (IHC), and mass spectrometry. For instance, antibodies to HDAC11 can be used to determine the presence and/or expression level of the protein in a sample either directly or indirectly e.g., using immunohistochemistry (IHC).

### Combination Therapies

In certain embodiments, the compounds and/or compositions contemplated within the disclosure are useful within the disclosure in combination with at least one additional agent useful for promoting weight loss. This additional compound may comprise compounds identified herein or compounds, e.g., commercially available compounds, known to promote weight loss.

Non-limiting examples of weight loss medications contemplated within the disclosure include orlistat, sibutramine, phendimetrazine tartrate, methamphetamine, IONAMIN^{™}, phentermine, fenfluramine, dexfenfluramine, chitosan, chromium picolinate, conjugated linoleic acid, green tea extract, guar gum, hoodia, a combination of topiramate and phentermine, a combination of bupropion and zonisamide, a combination of bupropion and naltrexone, a combination of phentermine and fluoxetine, a combination of phentermine and sertraline, a combination of phentermine and citalopram, a combination of phentermine and escitalopram, or a combination of phentermine and trazodone.

In certain embodiments, the compounds and/or compositions contemplated within the disclosure are useful within the disclosure in combination with at least one additional agent useful for treating or preventing metabolic syndrome or any disease or disorder included in and/or associated with metabolic syndrome (such as diabetes). This additional compound may comprise compounds identified herein or compounds, *e.g.,* commercially available compounds, known to treat, prevent or reduce the symptoms of metabolic syndrome or any disease or disorder associated with metabolic syndrome.

Non-limiting examples of antidiabetic medications contemplated within the disclosure include:
α-glucosidase inhibitors: inhibit upper GI enzymes (α-glucosidases) responsible for digesting carbohydrates, slowing absorption of glucose; also cause slower rise in postprandial blood glucose concentrations. Non-limiting examples: acarbose (Precose, Glucobay); miglitol (Glyset); voglibose (Vogseal, Volix, Basen);
lipase inhibitors: inhibit pancreatic and gastric lipases, blocking fat absorption. Non-limiting examples: orlistat (Xenical, Alli);
sulfonyl ureas: act as insulin secretagogues, triggering insulin release by interacting with the ATP-dependent potassium channel of the pancreatic β-cells. The net result is that more insulin is released at all blood glucose concentrations. They are the most commonly used drugs for treatment of patients with type 2 diabetes, but, since they trigger release of insulin itself, the combination of insulin & sulfonyl ureas is not common. Non-limiting examples: 1^{st} generation of sulfonyl ureas - acetohexamide, chlorpropamide (Diabinese), tolbutamide (Orinase), tolazamide; 2^{nd} generation of sulfonyl ureas - gliclazide (Diamicron R, Diamicron MR), glyburide or glibenclamide (Diabeta, Micronase, Glynase), glipizide (Glucotrol, Glucotrol XL), glimepiride (Amaryl), gliquidone (Glurenorm);
meglitinides: short-acting glucose-lowering drugs, acting by regulating ATP-dependent potassium channels in pancreatic β-cells like sulfonyl ureas; structurally different from sulfonylureas and act via different receptors as well. Non-limiting examples: mitiglinide (Glufast); nateglinide (Starlix); repaglinide (Prandix);
biguanides: reduce glucose release from the liver and increase glucose uptake by skeletal muscle. Metformin is the preferred initial treatment of type 2 diabetes, with good glycemic efficacy, absence of weight gain and hypoglycemia, general tolerability and low cost. The combination of metformin & insulin is generally associated with lower weight gain than insulin by itself or the combination of insulin & sulfonylureas. The triple combination of a sulfonyl urea, metformin and insulin glargine has been shown to have fewer adverse effects, fewer lipid profile problems and lower cost than the triple combination of a sulfonyl urea, metformin and rosiglitazone. Non-limiting examples: metformin (Glucophage); phenformin (DBI); buformin (Glybigid, Glybigidum);
thiazolidinediones: increase insulin sensitivity by acting on adipose, muscle and liver tissue to increase glucose utilization and decrease glucose production. The mechanism of action is not fully understood, but they seem to bind and activate one or more peroxisome proliferator-activated receptors (PPARs), regulating gene expression. Non-limiting examples: rosiglitazone (Avandia); pioglitazone (Actos); troglitazone (Rezulin); tesaglitazar (Pargluva);
pramlintide (Symlin): also known as islet amyloid polypeptide, is a synthetic analog of human amylin that slows gastric emptying and suppresses glucagon, reducing postprandial rises in blood glucose levels; approved by the FDA to lower blood sugar in type 1 diabetes patients;
incretin mimetics: these insulin secretagogues act as glucagon-like peptide-1 (GLP-1) membrane-receptor agonists. They act in a glucose-dependent manner, stimulating insulin secretion only when blood glucose levels are higher than normal. They also promote β-cell regeneration in animal models. Incretin mimetics decrease gastric motility and cause nausea. Non-limiting examples: exenatide, exedin-4 or AC2993 (Byetta); liraglutide, NN2211, or NNC 90-1170; it consists of a lipid conjugate of GLP-1, with high protein binding and a half-life of ~10 h in man;
DPP-IV inhibitors: affect glucose regulation, inhibiting degradation of GLP-1. They generally cause fewer problems with hypoglycemia or weight gain as compared to standard treatments. Non-limiting examples: sitagliptin (Januvia); sitagliptin & metformin (Janumet); vildagliptin (Galvus); vildagliptin & metformin (Eucreas);
SGLT2 inhibitors: they supress SGLT2 protein, causing excess glucose to be excreted from the body rather than reabsorbed. Non-limiting examples: dapaglifozin.

A synergistic effect may be calculated, for example, using suitable methods such as, for example, the Sigmoid-Eₘₐₓ equation (Holford & Scheiner, 1981, Clin. Pharmacokinet. 6: 429-453), the equation of Loewe additivity (Loewe & Muischnek, 1926, Arch. Exp. Pathol Pharmacol. 114: 313-326) and the median-effect equation (Chou & Talalay, 1984, Adv. Enzyme Regul. 22:27-55). Each equation referred to above may be applied to experimental data to generate a corresponding graph to aid in assessing the effects of the drug combination. The corresponding graphs associated with the equations referred to above are the concentration-effect curve, isobologram curve and combination index curve, respectively.

### Kits

Illustrated is a kit comprising a composition comprising an agent that specifically inhibits HDAC11 expression and/or activity, and an instruction manual. In certain embodiments, the agent is a monoclonal antibody specifically binding to HDAC11.

### Administration/Dosage/Formulations

The regimen of administration may affect what constitutes an effective amount. The therapeutic formulations may be administered to the subject either prior to or after the onset of a disease or disorder contemplated in the disclosure. Further, several divided dosages, as well as staggered dosages may be administered daily or sequentially, or the dose may be continuously infused, or may be a bolus injection. Further, the dosages of the therapeutic formulations may be proportionally increased or decreased as indicated by the exigencies of the therapeutic or prophylactic situation.

Administration of the compositions of the present disclosure to a patient, preferably a mammal, more preferably a human, may be carried out using known procedures, at dosages and for periods of time effective to treat a disease or disorder contemplated in the disclosure. An effective amount of the therapeutic compound necessary to achieve a therapeutic effect may vary according to factors such as the state of the disease or disorder in the patient; the age, sex, and weight of the patient; and the ability of the therapeutic compound to treat a disease or disorder contemplated in the disclosure. Dosage regimens may be adjusted to provide the optimum therapeutic response. For example, several divided doses may be administered daily or the dose may be proportionally reduced as indicated by the exigencies of the therapeutic situation. A non-limiting example of an effective dose range for a therapeutic compound of the disclosure is from about 0.1 and 5,000 mg/kg of body weight/per day. The pharmaceutical compositions useful for practicing the disclosure may be administered to deliver a dose of from 1 ng/kg/day and 100 mg/kg/day. In certain embodiments, the disclosure envisions administration of a dose which results in a concentration of the compound of the present disclosure from 1 µM and 10 µM in a mammal. One of ordinary skill in the art would be able to study the relevant factors and make the determination regarding the effective amount of the therapeutic compound without undue experimentation.

Actual dosage levels of the active ingredients in the pharmaceutical compositions of this disclosure may be varied so as to obtain an amount of the active ingredient that is effective to achieve the desired therapeutic response for a particular patient, composition, and mode of administration, without being toxic to the patient.

In particular, the selected dosage level depends upon a variety of factors including the activity of the particular compound employed, the time of administration, the rate of excretion of the compound, the duration of the treatment, other drugs, compounds or materials used in combination with the compound, the age, sex, weight, condition, general health and prior medical history of the patient being treated, and like factors well, known in the medical arts.

A medical doctor, *e.g.,* physician or veterinarian, having ordinary skill in the art may readily determine and prescribe the effective amount of the pharmaceutical composition required. For example, the physician or veterinarian could start doses of the compounds of the disclosure employed in the pharmaceutical composition at levels lower than that required in order to achieve the desired therapeutic effect and gradually increase the dosage until the desired effect is achieved.

In certain embodiments, the compositions of the disclosure are formulated using one or more pharmaceutically acceptable excipients or carriers. In other embodiments, the pharmaceutical compositions of the disclosure comprise a therapeutically effective amount of a compound of the disclosure and a pharmaceutically acceptable carrier.

In certain embodiments, the compositions of the disclosure are administered to the patient in dosages that range from one to five times per day or more. In other embodiments, the compositions of the disclosure are administered to the patient in range of dosages that include, but are not limited to, once every day, every two, days, every three days to once a week, and once every two weeks. It is readily apparent to one skilled in the art that the frequency of administration of the various combination compositions of the disclosure varies from individual to individual depending on many factors including, but not limited to, age, disease or disorder to be treated, gender, overall health, and other factors. Thus, the disclosure should not be construed to be limited to any particular dosage regime and the precise dosage and composition to be administered to any patient is determined by the attending physical taking all other factors about the patient into account.

Compounds of the disclosure for administration may be in the range of from about 1 µg to about 10,000 mg, about 20 µg to about 9,500 mg, about 40 µg to about 9,000 mg, about 75 µg to about 8,500 mg, about 150 µg to about 7,500 mg, about 200 µg to about 7,000 mg, about 3050 µg to about 6,000 mg, about 500 µg to about 5,000 mg, about 750 µg to about 4,000 mg, about 1 mg to about 3,000 mg, about 10 mg to about 2,500 mg, about 20 mg to about 2,000 mg, about 25 mg to about 1,500 mg, about 30 mg to about 1,000 mg, about 40 mg to about 900 mg, about 50 mg to about 800 mg, about 60 mg to about 750 mg, about 70 mg to about 600 mg, about 80 mg to about 500 mg, and any and all whole or partial increments therebetween.

In some embodiments, the dose of a compound of the disclosure is from about 1 mg and about 2,500 mg. In some embodiments, a dose of a compound of the disclosure used in compositions described herein is less than about 10,000 mg, or less than about 8,000 mg, or less than about 6,000 mg, or less than about 5,000 mg, or less than about 3,000 mg, or less than about 2,000 mg, or less than about 1,000 mg, or less than about 500 mg, or less than about 200 mg, or less than about 50 mg. Similarly, in some embodiments, a dose of a second compound as described herein is less than about 1,000 mg, or less than about 800 mg, or less than about 600 mg, or less than about 500 mg, or less than about 400 mg, or less than about 300 mg, or less than about 200 mg, or less than about 100 mg, or less than about 50 mg, or less than about 40 mg, or less than about 30 mg, or less than about 25 mg, or less than about 20 mg, or less than about 15 mg, or less than about 10 mg, or less than about 5 mg, or less than about 2 mg, or less than about 1 mg, or less than about 0.5 mg, and any and all whole or partial increments thereof.

In certain embodiments, the present disclosure is directed to a packaged pharmaceutical composition comprising a container holding a therapeutically effective amount of a compound of the disclosure, alone or in combination with a second pharmaceutical agent; and instructions for using the compound to treat, prevent, or reduce one or more symptoms of a disease or disorder contemplated in the disclosure.

Routes of administration of any of the compositions of the disclosure include oral, nasal, rectal, intravaginal, parenteral, buccal, sublingual or topical. The compounds for use in the disclosure may be formulated for administration by any suitable route, such as for oral or parenteral, for example, transdermal, transmucosal (*e.g.,* sublingual, lingual, (trans)buccal, (trans)urethral, vaginal (*e.g.,* trans- and perivaginally), (intra)nasal and (trans)rectal), intravesical, intrapulmonary, intraduodenal, intragastrical, intrathecal, subcutaneous, intramuscular, intradermal, intra-arterial, intravenous, intrabronchial, inhalation, and topical administration.

Suitable compositions and dosage forms include, for example, tablets, capsules, caplets, pills, gel caps, troches, dispersions, suspensions, solutions, syrups, granules, beads, transdermal patches, gels, powders, pellets, magmas, lozenges, creams, pastes, plasters, lotions, discs, suppositories, liquid sprays for nasal or oral administration, dry powder or aerosolized formulations for inhalation, compositions and formulations for intravesical administration and the like. It should be understood that the formulations and compositions that would be useful in the present disclosure are not limited to the particular formulations and compositions that are described herein.

### Oral Administration

For oral application, particularly suitable are tablets, dragees, liquids, drops, suppositories, or capsules, caplets and gelcaps. The compositions intended for oral use may be prepared according to any method known in the art and such compositions may contain one or more agents selected from the group consisting of inert, non-toxic pharmaceutically excipients that are suitable for the manufacture of tablets. Such excipients include, for example an inert diluent such as lactose; granulating and disintegrating agents such as cornstarch; binding agents such as starch; and lubricating agents such as magnesium stearate. The tablets may be uncoated or they may be coated by known techniques for elegance or to delay the release of the active ingredients. Formulations for oral use may also be presented as hard gelatin capsules wherein the active ingredient is mixed with an inert diluent.

For oral administration, the compounds of the disclosure may be in the form of tablets or capsules prepared by conventional means with pharmaceutically acceptable excipients such as binding agents (*e.g.,* polyvinylpyrrolidone, hydroxypropylcellulose or hydroxypropylmethylcellulose); fillers (*e.g.,* cornstarch, lactose, microcrystalline cellulose or calcium phosphate); lubricants (*e.g.,* magnesium stearate, talc, or silica); disintegrates (*e.g.,* sodium starch glycollate); or wetting agents (*e.g.,* sodium lauryl sulfate). If desired, the tablets may be coated using suitable methods and coating materials such as OPADRY^{™} film coating systems available from Colorcon, West Point, Pa. (*e.g.,* OPADRY^{™} OY Type, OYC Type, Organic Enteric OY-P Type, Aqueous Enteric OY-A Type, OY-PM Type and OPADRY^{™} White, 32K18400). Liquid preparation for oral administration may be in the form of solutions, syrups or suspensions. The liquid preparations may be prepared by conventional means with pharmaceutically acceptable additives such as suspending agents (*e.g.,* sorbitol syrup, methyl cellulose or hydrogenated edible fats); emulsifying agent (*e.g.,* lecithin or acacia); non-aqueous vehicles (*e.g.,* almond oil, oily esters or ethyl alcohol); and preservatives (*e.g.,* methyl or propyl p-hydroxy benzoates or sorbic acid).

The present disclosure also includes a multi-layer tablet comprising a layer providing for the delayed release of one or more compounds of the disclosure, and a further layer providing for the immediate release of a medication for treatment of a disease or disorder contemplated in the disclosure. Using a wax/pH-sensitive polymer mix, a gastric insoluble composition may be obtained in which the active ingredient is entrapped, ensuring its delayed release.

### Parenteral Administration

As used herein, "parenteral administration" of a pharmaceutical composition includes any route of administration characterized by physical breaching of a tissue of a subject and administration of the pharmaceutical composition through the breach in the tissue. Parenteral administration thus includes, but is not limited to, administration of a pharmaceutical composition by injection of the composition, by application of the composition through a surgical incision, by application of the composition through a tissue-penetrating non-surgical wound, and the like. In particular, parenteral administration is contemplated to include, but is not limited to, subcutaneous, intravenous, intraperitoneal, intramuscular, intrasternal injection, and kidney dialytic infusion techniques.

Formulations of a pharmaceutical composition suitable for parenteral administration comprise the active ingredient combined with a pharmaceutically acceptable carrier, such as sterile water or sterile isotonic saline. Such formulations may be prepared, packaged, or sold in a form suitable for bolus administration or for continuous administration. Injectable formulations may be prepared, packaged, or sold in unit dosage form, such as in ampules or in multidose containers containing a preservative. Formulations for parenteral administration include, but are not limited to, suspensions, solutions, emulsions in oily or aqueous vehicles, pastes, and implantable sustained-release or biodegradable formulations. Such formulations may further comprise one or more additional ingredients including, but not limited to, suspending, stabilizing, or dispersing agents. In certain embodiments of a formulation for parenteral administration, the active ingredient is provided in dry *(i.e.,* powder or granular) form for reconstitution with a suitable vehicle (*e.g.,* sterile pyrogen free water) prior to parenteral administration of the reconstituted composition.

The pharmaceutical compositions may be prepared, packaged, or sold in the form of a sterile injectable aqueous or oily suspension or solution. This suspension or solution may be formulated according to the known art, and may comprise, in addition to the active ingredient, additional ingredients such as the dispersing agents, wetting agents, or suspending agents described herein. Such sterile injectable formulations may be prepared using a non-toxic parenterally-acceptable diluent or solvent, such as water or 1,3-butanediol, for example. Other acceptable diluents and solvents include, but are not limited to, Ringer's solution, isotonic sodium chloride solution, and fixed oils such as synthetic mono- or di-glycerides. Other parentally-administrable formulations which are useful include those which comprise the active ingredient in microcrystalline form, in a liposomal preparation, or as a component of a biodegradable polymer system. Compositions for sustained release or implantation may comprise pharmaceutically acceptable polymeric or hydrophobic materials such as an emulsion, an ion exchange resin, a sparingly soluble polymer, or a sparingly soluble salt.

### Additional Administration Forms

Additional dosage forms of this disclosure include dosage forms as described in U.S. Patents Nos. 6,340,475; 6,488,962; 6,451,808; 5,972,389; 5,582,837; and 5,007,790. Additional dosage forms of this disclosure also include dosage forms as described in U.S. Patent Applications Nos. 20030147952; 20030104062; 20030104053; 20030044466; 20030039688; and 20020051820. Additional dosage forms of this disclosure also include dosage forms as described in PCT Applications Nos. WO 03/35041; WO 03/35040; WO 03/35029; WO 03/35177; WO 03/35039; WO 02/96404; WO 02/32416; WO 01/97783; WO 01/56544; WO 01/32217; WO 98/55107; WO 98/11879; WO 97/47285; WO 93/18755; and WO 90/11757.

### Controlled Release Formulations and Drug Delivery Systems

In certain embodiments, the formulations of the present disclosure may be, but are not limited to, short-term, rapid-offset, as well as controlled, for example, sustained release, delayed release and pulsatile release formulations.

The term sustained release is used in its conventional sense to refer to a drug formulation that provides for gradual release of a drug over an extended period of time, and that may, although not necessarily, result in substantially constant blood levels of a drug over an extended time period. The period of time may be as long as a month or more and should be a release which is longer that the same amount of agent administered in bolus form.

For sustained release, the compounds may be formulated with a suitable polymer or hydrophobic material that provides sustained release properties to the compounds. As such, the compounds for use the method of the disclosure may be administered in the form of microparticles, for example, by injection or in the form of wafers or discs by implantation.

In certain embodiments, the compounds of the disclosure are administered to a patient, alone or in combination with another pharmaceutical agent, using a sustained release formulation.

The term delayed release is used herein in its conventional sense to refer to a drug formulation that provides for an initial release of the drug after some delay following drug administration and that may, although not necessarily, includes a delay of from about 10 minutes up to about 12 hours.

The term pulsatile release is used herein in its conventional sense to refer to a drug formulation that provides release of the drug in such a way as to produce pulsed plasma profiles of the drug after drug administration.

The term immediate release is used in its conventional sense to refer to a drug formulation that provides for release of the drug immediately after drug administration.

As used herein, short-term refers to any period of time up to and including about 8 hours, about 7 hours, about 6 hours, about 5 hours, about 4 hours, about 3 hours, about 2 hours, about 1 hour, about 40 minutes, about 20 minutes, or about 10 minutes and any or all whole or partial increments thereof after drug administration after drug administration.

As used herein, rapid-offset refers to any period of time up to and including about 8 hours, about 7 hours, about 6 hours, about 5 hours, about 4 hours, about 3 hours, about 2 hours, about 1 hour, about 40 minutes, about 20 minutes, or about 10 minutes, and any and all whole or partial increments thereof after drug administration.

### Dosing

The therapeutically effective amount or dose of a compound of the present disclosure depends on the age, sex and weight of the patient, the current medical condition of the patient and the progression of a disease or disorder contemplated in the disclosure. The skilled artisan is able to determine appropriate dosages depending on these and other factors.

A suitable dose of a compound of the present disclosure may be in the range of from about 0.01 mg to about 5,000 mg per day, such as from about 0.1 mg to about 1,000 mg, for example, from about 1 mg to about 500 mg, such as about 5 mg to about 250 mg per day. The dose may be administered in a single dosage or in multiple dosages, for example from 1 to 4 or more times per day. When multiple dosages are used, the amount of each dosage may be the same or different. For example, a dose of 1 mg per day may be administered as two 0.5 mg doses, with about a 12-hour interval between doses.

It is understood that the amount of compound dosed per day may be administered, in non-limiting examples, every day, every other day, every 2 days, every 3 days, every 4 days, or every 5 days. For example, with every other day administration, a 5 mg per day dose may be initiated on Monday with a first subsequent 5 mg per day dose administered on Wednesday, a second subsequent 5 mg per day dose administered on Friday, and so on.

In the case wherein the patient's status does improve, upon the doctor's discretion the administration of the compound of the disclosure is optionally given continuously; alternatively, the dose of drug being administered is temporarily reduced or temporarily suspended for a certain length of time (*i.e.,* a "drug holiday"). The length of the drug holiday optionally varies between 2 days and 1 year, including by way of example only, 2 days, 3 days, 4 days, 5 days, 6 days, 7 days, 10 days, 12 days, 15 days, 20 days, 28 days, 35 days, 50 days, 70 days, 100 days, 120 days, 150 days, 180 days, 200 days, 250 days, 280 days, 300 days, 320 days, 350 days, or 365 days. The dose reduction during a drug holiday includes from 10%-100%, including, by way of example only, 10%, 15%, 20%, 25%, 30%, 35%, 40%, 45%, 50%, 55%, 60%, 65%, 70%, 75%, 80%, 85%, 90%, 95%, or 100%.

Once improvement of the patient's conditions has occurred, a maintenance dose is administered if necessary. Subsequently, the dosage or the frequency of administration, or both, is reduced, as a function of the disease or disorder, to a level at which the improved disease is retained

Toxicity and therapeutic efficacy of such therapeutic regimens are optionally determined in cell cultures or experimental animals, including, but not limited to, the determination of the LD₅₀ (the dose lethal to 50% of the population) and the ED₅₀ (the dose therapeutically effective in 50% of the population). The dose ratio between the toxic and therapeutic effects is the therapeutic index, which is expressed as the ratio between LD₅₀ and ED₅₀. The data obtained from cell culture assays and animal studies are optionally used in formulating a range of dosage for use in humans. The dosage of such compounds lies preferably within a range of circulating concentrations that include the ED50 with minimal toxicity. The dosage optionally varies within this range depending upon the dosage form employed and the route of administration utilized.

Those skilled in the art will recognize, or be able to ascertain using no more than routine experimentation, numerous equivalents to the specific procedures, embodiments, claims, and examples described herein. Such equivalents were considered to be within the scope of this disclosure and covered by the claims appended hereto. For example, it should be understood, that modifications in reaction conditions, including but not limited to reaction times, reaction size/volume, and experimental reagents, such as solvents, catalysts, pressures, atmospheric conditions, e.g., nitrogen atmosphere, and reducing/oxidizing agents, with art-recognized alternatives and using no more than routine experimentation, are within the scope of the present application.

It is to be understood that wherever values and ranges are provided herein, all values and ranges encompassed by these values and ranges, are meant to be encompassed within the scope of the present disclosure. Moreover, all values that fall within these ranges, as well as the upper or lower limits of a range of values, are also contemplated by the present application.

The following examples further illustrate aspects of the present disclosure.

### EXAMPLES

The disclosure is now described with reference to the following Examples. These Examples are provided for the purpose of illustration only, and the disclosure is not limited to these Examples, but rather encompasses all variations that are evident as a result of the teachings provided herein.

### Materials and Methods

Unless otherwise noted, all cell lines, starting materials and reagents were obtained from commercial suppliers and used without purification.

### Example 1:

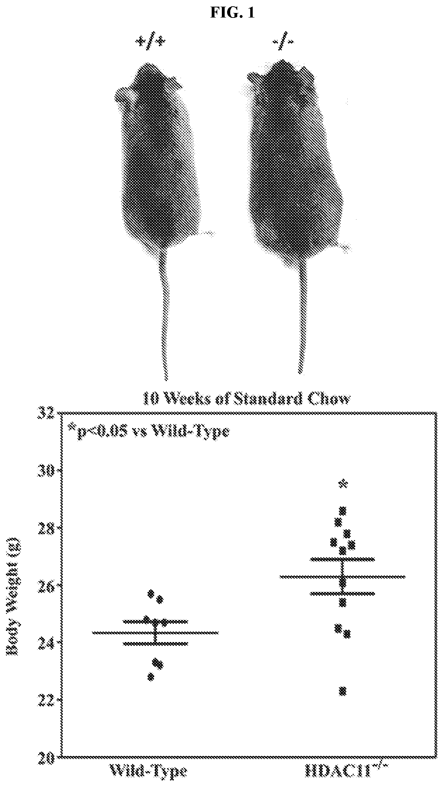

As an initial experiment, HDAC11 knockout (indicated as HDAC11 KO, or HDAC11^{-/-}) mice were tested for their general ability to gain weight. FIG. 1 illustrates the finding that 10-week-old HDAC11 KO mice were larger and had increased weight as compared to wild-type (WT) mice, all of which fed on standard chow diet.

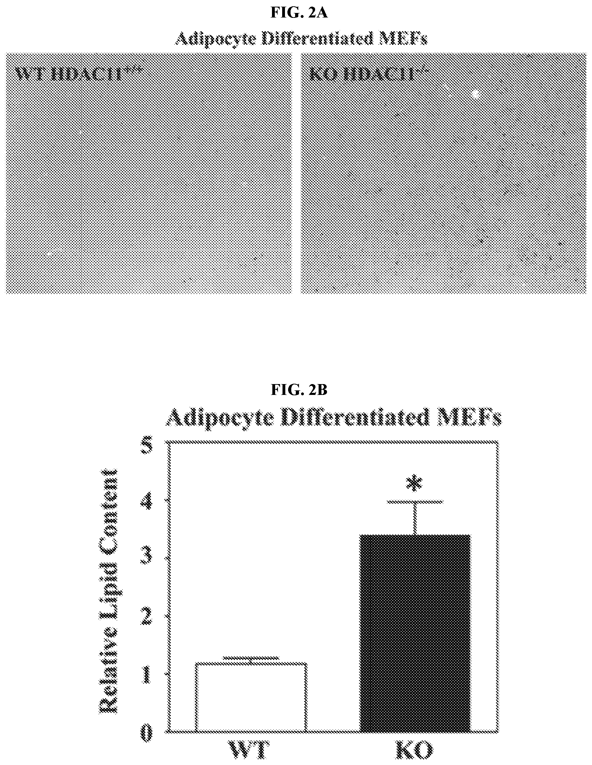

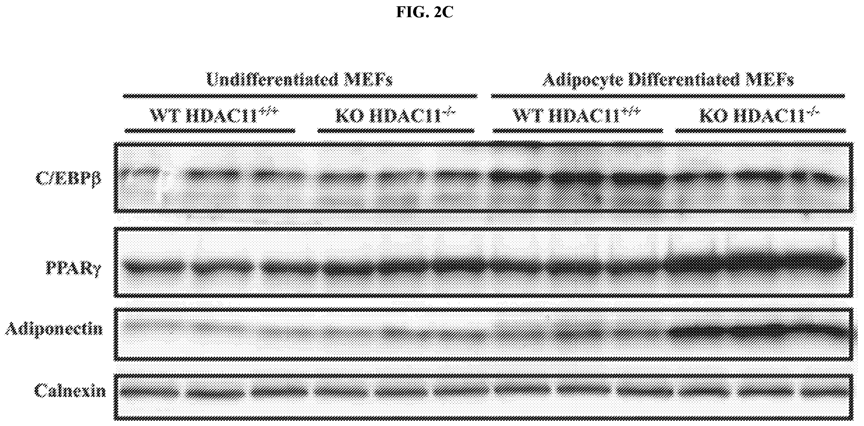

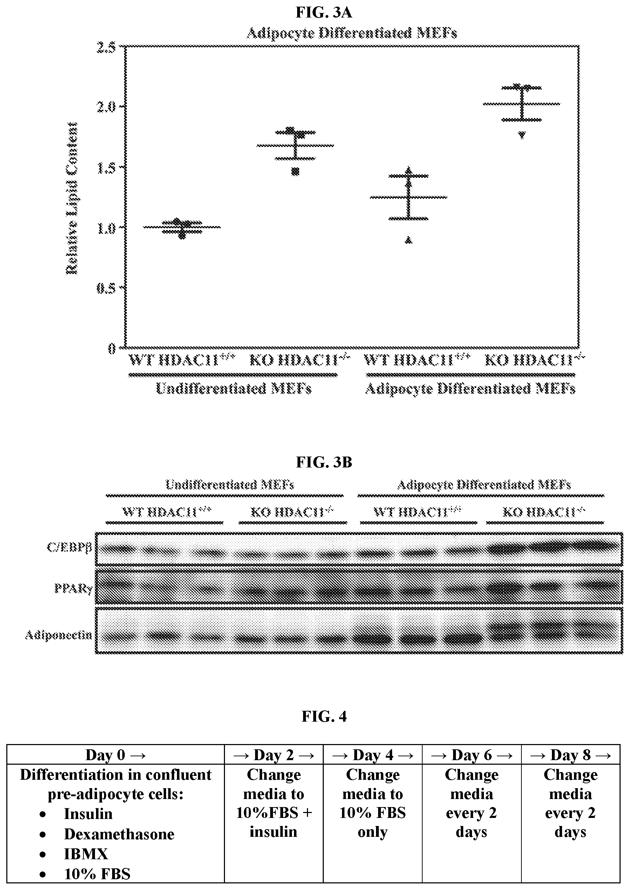

In order to obtain further insight into the effect of HADC11 on adipogenesis, HDAC11 KO mouse embryonic fibroblasts (MEFs) were compared to the corresponding WT MEFs. The HDAC11 KO MEFs underwent increased adipogenic differentiation (FIG. 2A; note the stained lipid droplets, which are more prominent on the right panel), which was quantified as being at least 100% higher than in the WT MEFs (FIG. 2B). Further, the adipocyte differentiated HDAC11 KO MEFs showed increased expression of known adipogenesis markers, such as PPARγ and adiponectin (FIG. 2C). Increased lipid content over the corresponding WT cells was observed for both the undifferentiated and adipocyte-differentiated HDAC11 KO MEFs (FIGs. 3A-3B).

### Example 2:

In order to assess adipogenesis, a cell-based assay using 3T3-L1 pre-adipocyte cell line was established (FIG. 4). On Day 0, differentiation in confluent pre-adipocyte cells was implemented by contacting the cells with a medium comprising insulin, dexamethasome, IBMX (3-isobutyl-1-methylxanthine), and 10% FBS (fetal bovine serum). On Day 2, the medium was exchanged for a medium comprising 10% FBS and insulin. On Day 4, the medium was exchanged for a medium comprising 10% FBS. Starting Day 6 and from then on, the medium was changed every 2 days.

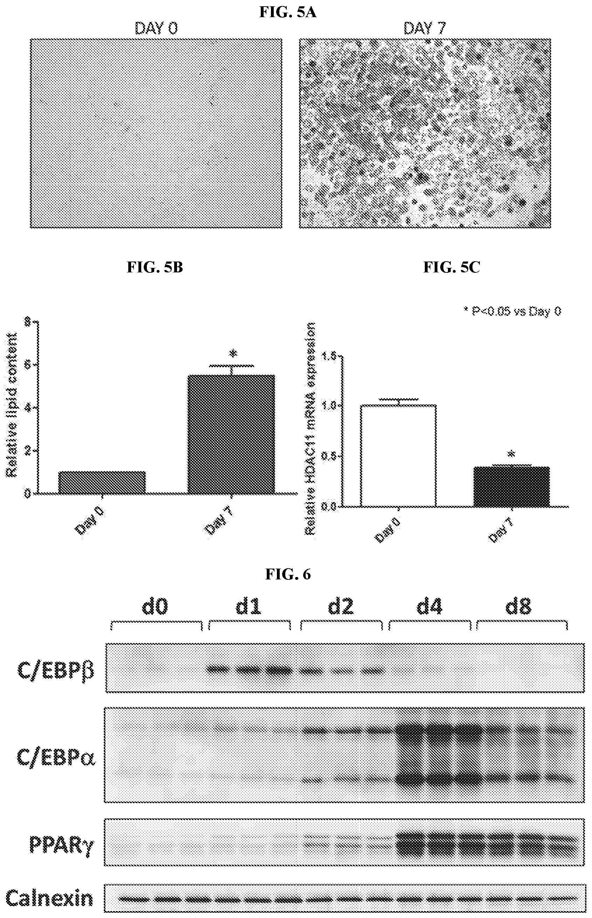

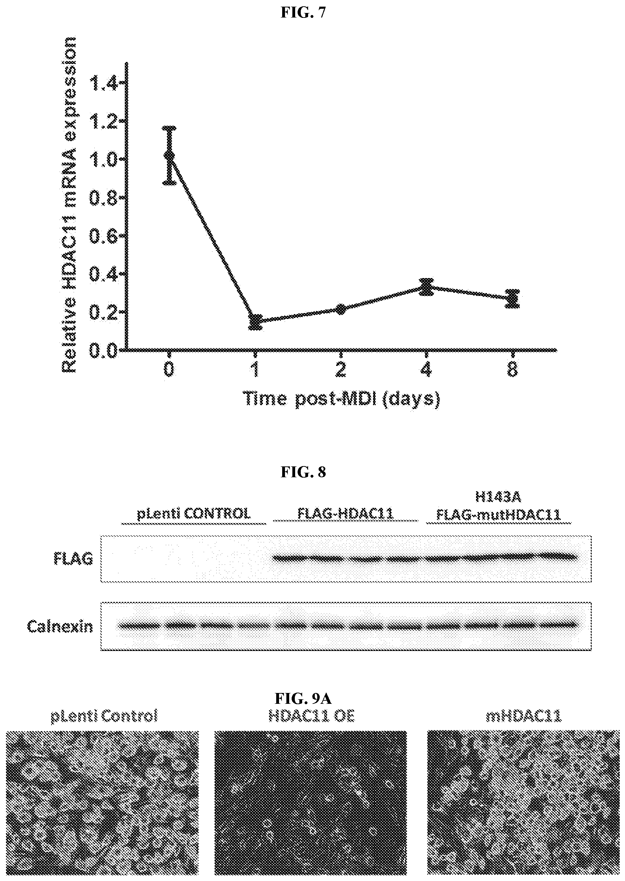

The adipocyte differentiated 3T3-L1 cells (in Day 0 and Day 7) were stained with oil-red O (a lysochrome diazo dye also known as Solvent Red 27, Sudan red 5B, or 1-(2,5-dimethyl-4-(2,5-dimethylphenyl) phenyldiazenyl) azonapthalen-2-ol), which indicated time-dependent formation of lipid droplets in those cells (FIG. 5A). Quantitation of the lipid content of the stained cells (depicted in FIG. 5A) is illustrated in FIG. 5B. As demonstrated in FIG. 5C, the level of HDAC11 mRNA in differentiated cells decreased markedly from Day 0 to Day 7. Consistently, HDAC1 was found to be downregulated during the course of differentiation in 3T3-L1 cells (FIG. 7), with a sharp decrease between Days 0 and 1.

The expression of various adipogenic markers during 3T3-L1 differentiation was analyzed as a function of time (FIG. 6). C/EBPβ accumulated transiently on Days 1-2, while C/EBPα and PPARγ had higher concentrations on Day 4, decreasing in concentration towards Day 8.

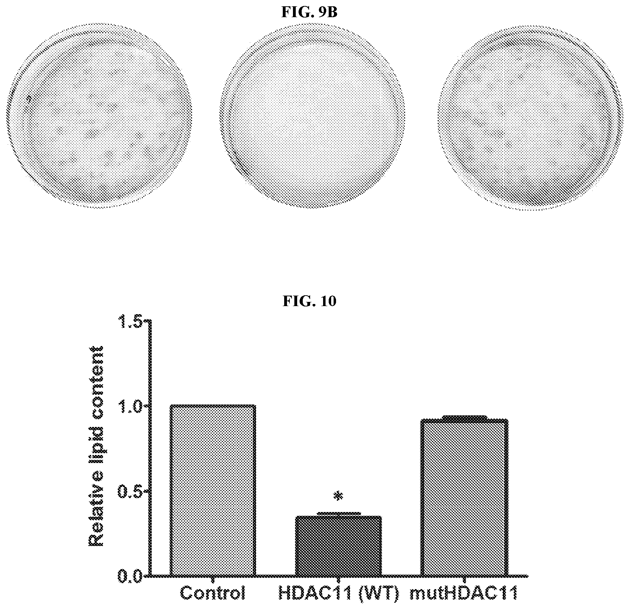

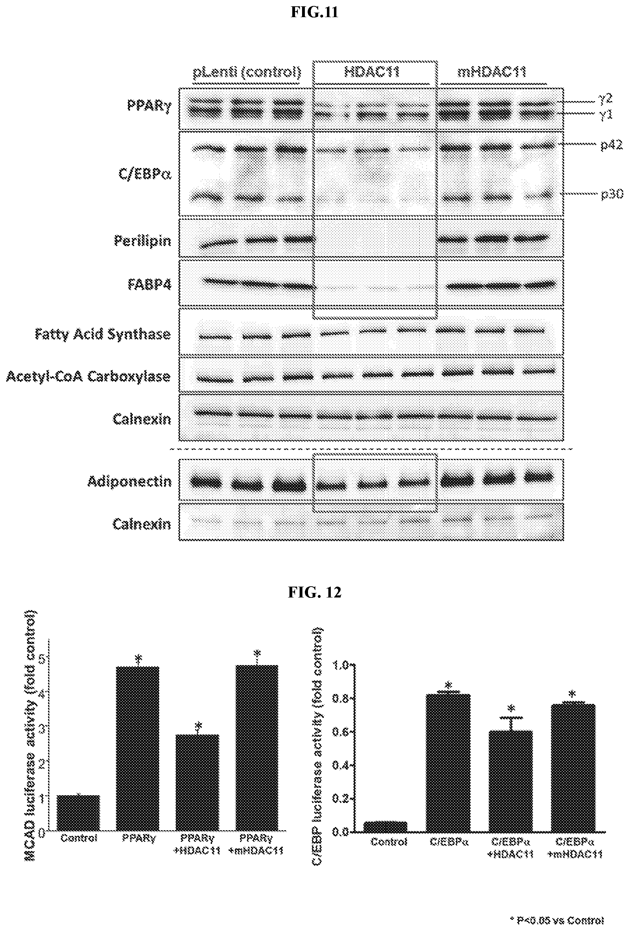

A system for overexpression of HDAC11 in 3T3-L1 pre-adipocytes was developed using lentiviral vectors. As demonstrated by western blotting (FIG. 8), FLAG-tagged HDAC11 was efficiently overexpressed in 3T3-L1 cells using the lentiviral vectors, including cells containing mutant HDAC11 H143A (right lanes), which is catalytically impaired. The results of HDAC11 overexpression in these cells was evidenced using both phase contrast images (FIG. 9A) and Oil Red O staining for lipid droplets (FIG. 9B): HDAC11 overexpression reduced lipid accumulation during adipogenesis (FIG. 10), which was not observed with mutated HDAC11 H143A. In fact, ectopic expression of HDAC11 in these cells had the effect of blunting adipogenesis, repressing the expression of selected adipogenic markers (such as C/EBPα, perilipin, FABP4 and adiponectin; FIG. 11). HDAC11 overexpression was further found to attenuate promoter transcription for adipogenic markers PPARγ and C/EBPα (FIG. 12).

### Example 3:

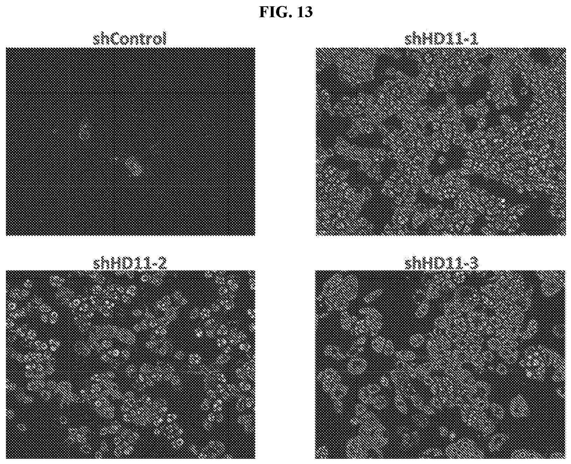

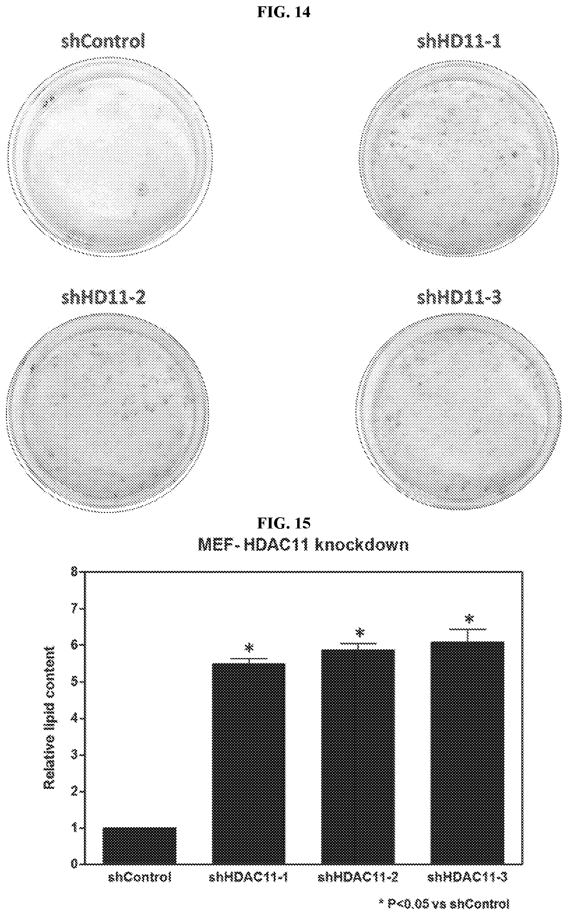

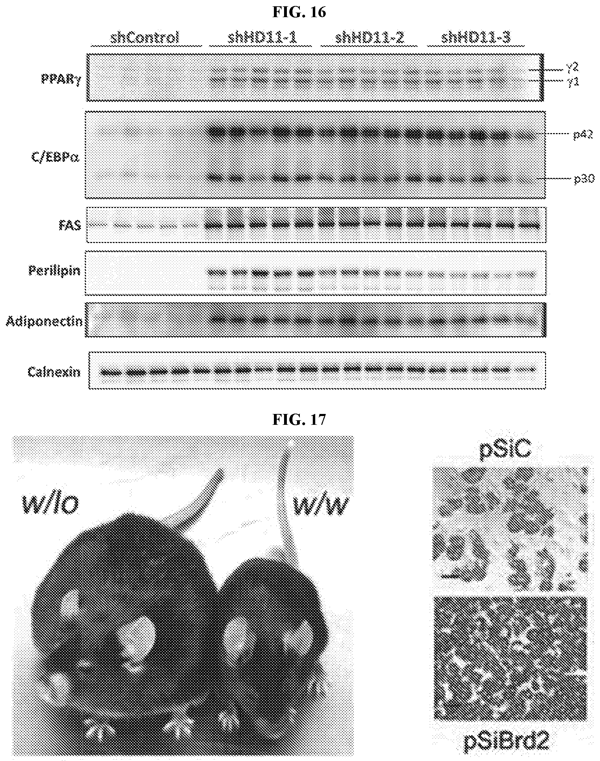

The effects on adipogenesis from the loss of HDAC11 function in MEFs was evaluated. HDAC11 knockdown was found to enhance adipogenic differentiation in those cells, as evidenced using phase contrast imaging of lipid droplets in MEFs on Day 6 of post-differentiation induction (FIGs. 13 and 16). This result was confirmed using Oil red O staining of lipid accumulation (FIG. 14). Further, HDAC11 knockdown also augmented lipid accumulation during adipogenesis in MEFs by as much as 450% (FIG. 15).

### Example 4:

BRD2, formerly known as "RING3," is a close homologue of TAF (TATA-box-binding protein-associated factor)II250, a basal transcription co-activator. BRD2 is one of the protein factors that regulate gene transcription by controlling the activity of transcription complexes, interpreting the histone code and remodeling chromatin. BRD2 belongs to the BET (bromodomain and extraterminal domain) subfamily of proteins that harbor two bromodomains. Bromodomain proteins serve as adaptor or scaffolding modules that recruit transcription regulatory factors to chromatin to form protein complexes that regulate gene transcription in response to signal transduction.

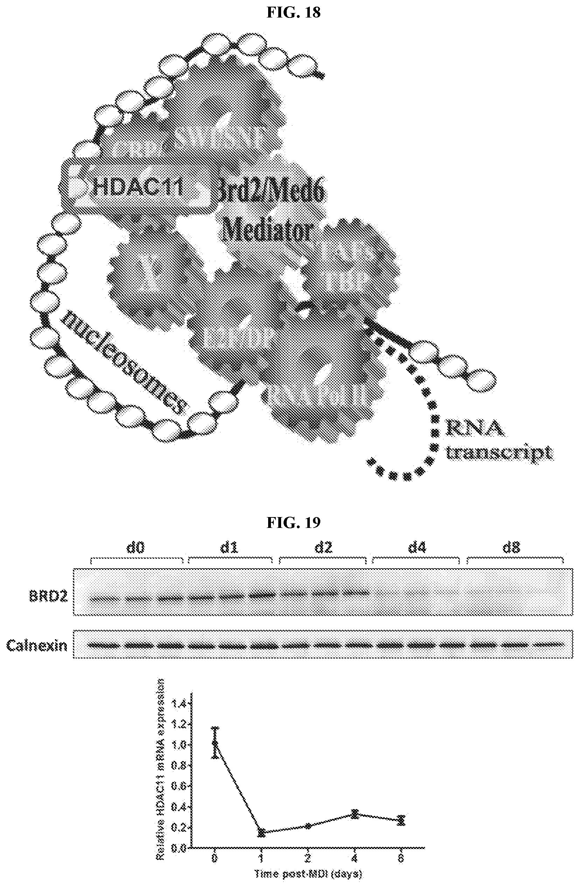

BRD2 is a signal transducer and a nuclear-localized serine/threonine kinase, and is ubiquitously expressed in mammalian tissues. BRD2 binds not only transcriptional activators like E2F proteins, but also co-activator TAFs, members of the SWI/SNF complex, and HDACs to regulate the expression of diverse genes (FIG. 18). As with the SWI/SNF complex with which it associates, BRD2 can function as either a transcriptional co-activator or co-repressor, depending on the specific promoter and cellular context.

Whole body disruption of *Brd2,* an unusual MHC gene, causes lifelong severe obesity in mice with pancreatic islet expansion, hyperinsulinaemia, hepatosteatosis and elevated pro-inflammatory cytokines, but, surprisingly, enhanced glucose tolerance, elevated adiponectin, increased weight of brown adipose tissue, heat production and expression of mitochondrial uncoupling proteins in brown adipose tissue, reduced macrophage infiltration in white adipose tissue, and lowered blood glucose, leading to an improved metabolic profile and avoiding eventual Type 2 diabetes (FIG. 17). In 3T3-L1 pre-adipocytes, BRD2 normally co-represses PPAR-γ (peroxisome-proliferator-activated receptor-γ) and inhibits adipogenesis. BRD2 knockdown protects 3T3-L1 adipocytes from TNF-α (tumor necrosis factor-α) induced insulin resistance, thereby decoupling inflammation from insulin resistance. Hypomorphic BRD2 shifts energy balance toward storage without causing glucose intolerance.

The functional role of the HDAC11-BRD2 interaction is unknown at this time.

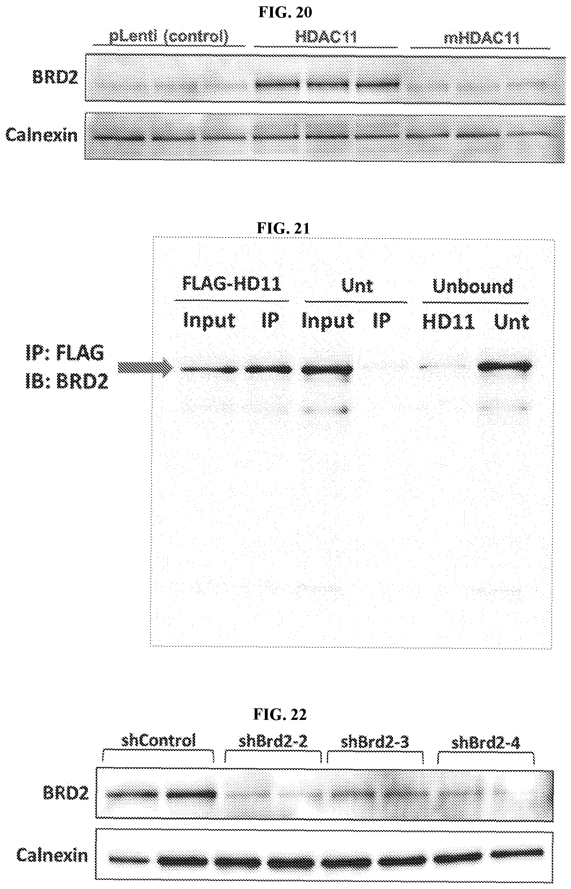

As illustrated in FIG. 19, BRD2 expression was down-regulated during adipogenesis in 3T3-L1 cells (FIG. 19). In fact, during differentiation of 3T3-L1 cells, BRD2 expression (FIG. 19) nearly paralleled that of HDAC11 (FIG. 7). BRD2 expression was enhanced in response to ectopic expression of HDAC11 (FIG. 20), indicating that HDAC11 overexpression drives BRD2 expression in 3T3-L1 cells. Co-immunoprecipitation in 293A cells indicated that HDAC11 and BRD2 physically interact to form a complex (FIG. 21).

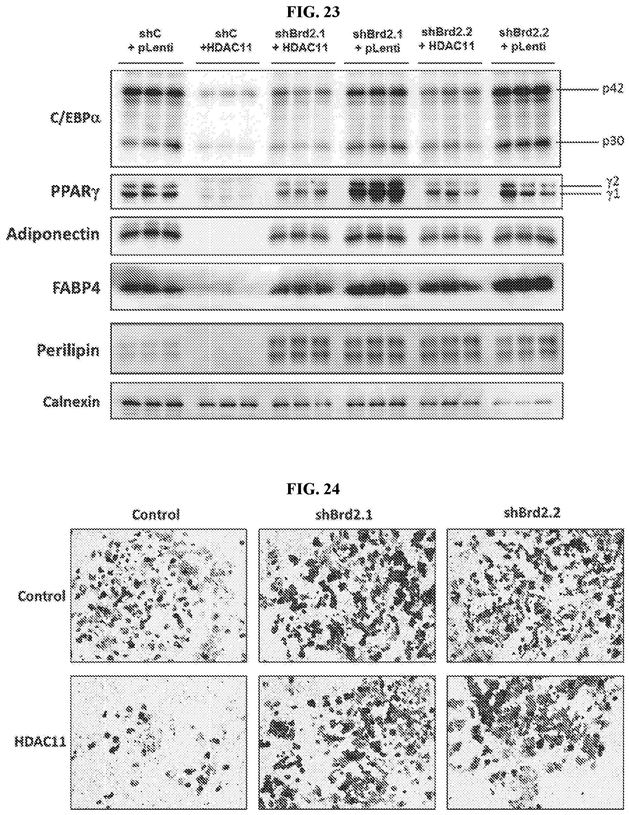

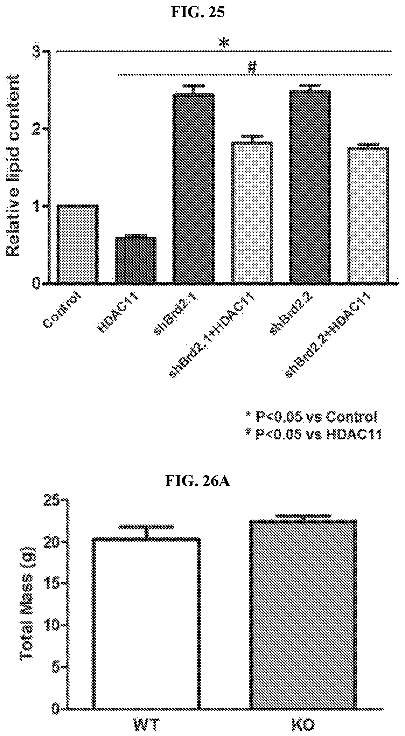

BRD2 was efficiently knocked down in 3T3-L1 pre-adipocytes using lentiviral delivery, as demonstrated using western blotting (FIG. 22). As demonstrated for 3T3-L1 cells treated with lentiviral expression vectors, *Brd2* gene knockdown partially rescued the repressive effects of HDAC11 on adipogenesis (note the enhancement of selected adipogenic markers in FIG. 23 for the BRD2 knockdown cells). This effect is further observed by visually inspecting stained lipid droplets (FIG. 24) and quantitating lipid content (FIG. 25) in these cells.

### Example 5:

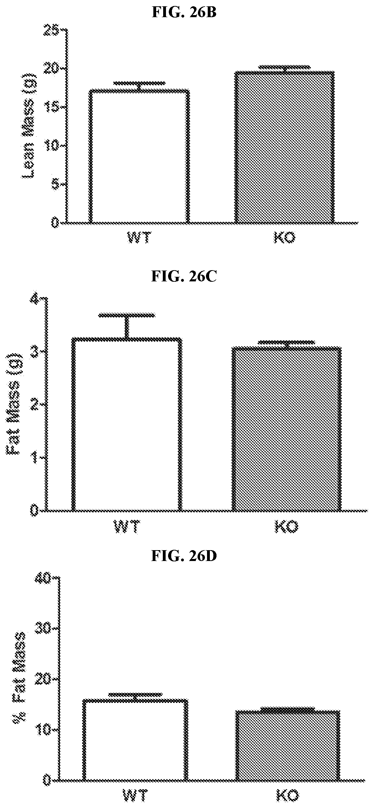

The phenotype of HDAC11 knockout mice was evaluated. For that, bone densitometry (DEXA) scans were performed on 10-11 week-old male WT and HDAC11 KO mice (n=4). The results indicated that HDAC11 KO mice have increased body mass and lean mass, but similar fat mass compared to WT litter mates (FIG. 26).

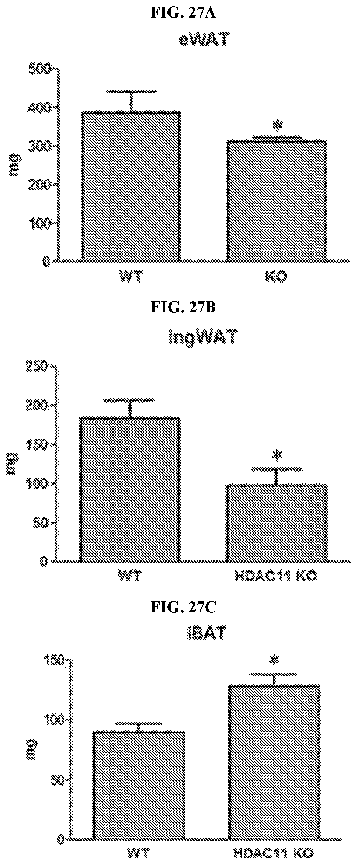

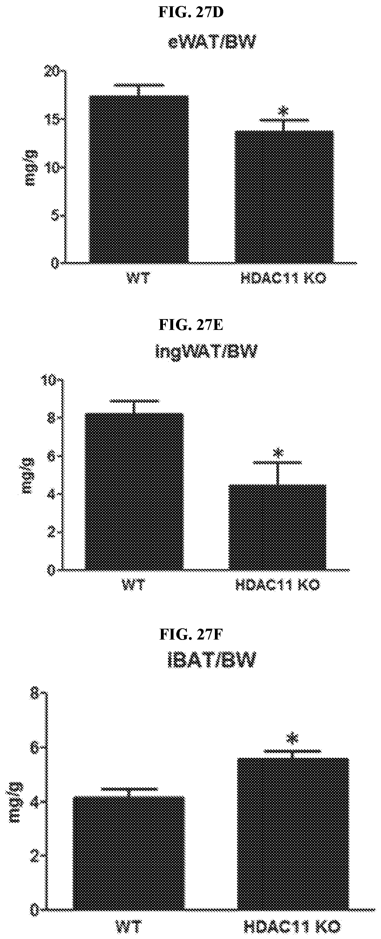

The 10-11 week-old male WT and HDAC11 mice (n=4) were euthanized, and three different fat depots from each mouse were harvested and weighted. Analysis of the HDAC11 KO mice showed that they had increased brown fat depots compared to the WT animals (FIG. 27).

## Claims

1. A composition that inhibits HDAC11 expression and/or activity, or induces HDAC11 degradation for use in (i) a method of treating, reverting, and/or preventing obesity, hyperlipidemia, type II diabetes, metabolic syndrome or insulin resistance in a subject in need thereof, wherein the composition comprises at least one agent selected from the group consisting of an antibody, siRNA, ribozyme, antisense RNA, modified antisense RNA, small molecule, and any combinations thereof.

2. Non-therapeutic use of a composition that inhibits HDAC11 expression and/or activity, or induces HDAC11 degradation for promoting weight loss, and/or ameliorating or preventing weight gain, wherein the composition comprises at least one agent selected from the group consisting of an antibody, siRNA, ribozyme, antisense RNA, modified antisense RNA, small molecule, and any combinations thereof.

3. The composition for use according to claim 1 or the use of claim 2, wherein the subject has at least one condition selected from the group consisting of elevated blood pressure, elevated fasting plasma glucose, high serum triglyceride levels, and low high-density lipoprotein (HDL) levels.

4. The composition for use according to claim 1, wherein the subject has type II diabetes.

5. The composition for use according to claim 1 or the use of claim 2, wherein the antibody comprises an antibody selected from the group consisting of a polyclonal antibody, monoclonal antibody, humanized antibody, synthetic antibody, heavy chain antibody, human antibody, biologically active fragment of an antibody, and any combinations thereof.

6. The composition for use or the use according to any one of claim 1-5, wherein the composition is administered to the subject by an inhalational, oral, rectal, vaginal, parenteral, topical, transdermal, pulmonary, intranasal, buccal, ophthalmic, intrathecal, intracranial, or intravenous route of administration.

7. The composition for use or the use according to any one of claim 1-6, wherein the composition is co-administered with at least one additional agent that treats and/or prevents metabolic syndrome, or promotes weight loss.

8. The composition for use or the use according to claim 7, wherein that at least one additional agent comprises a weight loss promoter or anti-diabetic medicament.

9. The composition for use or the use according to any of claims 1-8, wherein the subject is a mammal.

10. The composition for use or the use according to claim 9, wherein the mammal is human.

## Patentansprüche

1. Eine Zusammensetzung, die die Expression und/oder Aktivität von HDAC11 hemmt oder den Abbau von HDAC11 induziert, zur Verwendung in (i) einem Verfahren zur Behandlung, Umkehrung und/oder Vorbeugung von Adipositas, Hyperlipidämie, Typ-II-Diabetes, metabolischem Syndrom oder Insulinresistenz bei einem Patienten, der dies benötigt, wobei die Zusammensetzung mindestens einen Wirkstoff umfasst, der aus der Gruppe ausgewählt ist, die aus einem Antikörper, einer siRNA, einem Ribozym, einer Antisense-RNA, modifizierter Antisense-RNA, einem kleinen Molekül und beliebigen Kombinationen davon besteht.

2. Nicht-therapeutische Verwendung einer Zusammensetzung, die die Expression und/oder Aktivität von HDAC11 hemmt oder den Abbau von HDAC11 induziert, um die Gewichtsabnahme zu fördern und/oder die Gewichtszunahme zu verbessern oder zu verhindern, wobei die Zusammensetzung mindestens einen Wirkstoff umfasst, der aus der Gruppe ausgewählt ist, die aus einem Antikörper, einer siRNA, einem Ribozym, einer Antisense-RNA, modifizierter Antisense-RNA, einem kleinen Molekül und beliebigen Kombinationen davon besteht..

3. Die Zusammensetzung zur Verwendung gemäß Anspruch 1 oder die Verwendung gemäß Anspruch 2, wobei der Patient mindestens einen Zustand aufweist, der aus der Gruppe ausgewählt ist, die aus erhöhtem Blutdruck, erhöhtem Nüchtemplasmaglukosespiegel, hohen Serumtriglyceridspiegeln und niedrigen High-Density-Lipoprotein (HDL)-Spiegeln besteht.

4. Die Zusammensetzung zur Verwendung gemäß Anspruch 1, wobei der Patient Typ-II-Diabetes hat.

5. Die Zusammensetzung zur Verwendung gemäß Anspruch 1 oder die Verwendung gemäß Anspruch 2, wobei der Antikörper einen Antikörper umfasst, der aus der Gruppe ausgewählt ist, die aus einem polyklonalen Antikörper, einem monoklonalen Antikörper, einem humanisierten Antikörper, einem synthetischen Antikörper, einem Antikörper mit schwerer Kette, einem humanen Antikörper, einem biologisch aktiven Fragment eines Antikörpers und beliebigen Kombinationen davon besteht.

6. Die Zusammensetzung zur Verwendung oder die Verwendung gemäß einem der Ansprüche 1 bis 5, wobei die Zusammensetzung dem Patienten durch inhalative, orale, rektale, vaginale, parenterale, topische, transdermale, pulmonale, intranasale, bukkale, ophthalmische, intrathekale, intrakranielle oder intravenöse Verabreichung verabreicht wird.

7. Die Zusammensetzung zur Verwendung oder die Verwendung gemäß einem der Ansprüche 1 bis 6, wobei die Zusammensetzung zusammen mit mindestens einem zusätzlichen Wirkstoff verabreicht wird, der das metabolische Syndrom behandelt und/oder verhindert oder die Gewichtsabnahme fördert.

8. Die Zusammensetzung zur Verwendung oder die Verwendung gemäß Anspruch 7, wobei der mindestens eine zusätzliche Wirkstoff einen Wirkstoff zur Förderung der Gewichtsabnahme oder ein Antidiabetikum umfasst.

9. Die Zusammensetzung zur Verwendung oder die Verwendung gemäß einem der Ansprüche 1 bis 8, wobei der Patient ein Säugetier ist.

10. Die Zusammensetzung zur Verwendung oder die Verwendung gemäß Anspruch 9, wobei das Säugetier ein Mensch ist.

## Revendications

1. Composition qui inhibe l'expression et/ou l'activité de HDAC11 ou qui induit une dégradation de HDAC11 pour une utilisation (i) au niveau d'un procédé de traitement, de réversion et/ou de prévention de l'obésité, de l'hyperlipidémie, du diabète de type II, du syndrome métabolique ou de la résistance à l'insuline chez un sujet qui en a besoin, dans laquelle la composition comprend au moins un agent qui est sélectionné parmi le groupe qui est constitué par un anticorps, des petits acides ribonucléiques/ARN interférents ou siARN, un ribozyme, de l'ARN antisens, de l'ARN antisens modifié, des petites molécules et de quelconques combinaisons afférentes.

2. Utilisation non thérapeutique d'une combinaison qui inhibe l'expression et/ou l'activité de HDAC11 ou qui induit une dégradation de HDAC11 pour favoriser la perte de poids et/ou améliorer ou prévenir la prise de poids, dans laquelle la composition comprend au moins un agent qui est sélectionné parmi le groupe qui est constitué par un anticorps, des petits acides ribonucléiques/ARN interférents ou siARN, un ribozyme, de l'ARN antisens, de l'ARN antisens modifié, des petites molécules et de quelconques combinaisons afférentes.

3. Composition pour une utilisation selon la revendication 1 ou utilisation selon la revendication 2, dans laquelle le sujet présente au moins une pathologie qui est sélectionnée parmi le groupe qui est constitué par une pression sanguine élevée, un taux élevé de glucose plasmatique à jeun, des taux élevés de triglycérides sériques et des niveaux faibles de lipoprotéines de haute densité (HDL).

4. Composition pour une utilisation selon la revendication 1, dans laquelle le sujet présente un diabète de type II.

5. Composition pour une utilisation selon la revendication 1 ou utilisation selon la revendication 2, dans laquelle l'anticorps comprend un anticorps qui est sélectionné parmi le groupe qui est constitué par un anticorps polyclonal, un anticorps monoclonal, un anticorps humanisé, un anticorps synthétique, un anticorps à chaîne lourde, un anticorps humain, un fragment biologiquement actif d'un anticorps et de quelconques combinaisons afférentes.

6. Composition pour une utilisation ou utilisation selon l'une quelconque des revendications 1 à 5, dans laquelle la composition est administrée au sujet au moyen d'une voie d'administration par inhalation, orale, rectale, vaginale, parentérale, topique, transdermique, pulmonaire, intranasale, buccale, ophtalmique, intrathécale, intracrânienne ou intraveineuse.

7. Composition pour une utilisation ou utilisation selon l'une quelconque des revendications 1 à 6, dans laquelle la composition est co-administrée avec au moins un agent additionnel qui traite et/ou prévient le syndrome métabolique ou qui favorise la perte de poids.

8. Composition pour une utilisation ou utilisation selon la revendication 7, dans laquelle l'au moins un agent additionnel comprend un agent favorisant la perte de poids ou un médicament antidiabétique.

9. Composition pour une utilisation ou utilisation selon l'une quelconque des revendications 1 à 8, dans laquelle le sujet est un mammifère.

10. Composition pour une utilisation ou utilisation selon la revendication 9, dans laquelle le mammifère est un être humain.
